# FASCICULE DE BREVET EUROPEEN

(11) **EP 2 812 020 B1**
(45) Date de publication et mention de la délivrance du brevet: **23.05.2018**
(21) Numéro de dépôt: 13706643.7
(22) Date de dépôt: 06.02.2013
(51) Int. Cl.: A61K 38/45, A61K 39/002, A61K 39/00, C12N 9/10, A61P 29/00, A61P 37/02

(54) **PROTEINES 28KDA GST PROVENANT DE SCHISTOSOMES POUR LEUR UTILISATION DANS LE TRAITEMENT DES MALADIES INFLAMMATOIRES AUTO-IMMUNES ENGENDRANT UNE REPONSE DE TYPE TH1 ET/OU TH17**
28 KDA-GST-PROTEINE AUS SCHISTOSOMA ZUR BEHANDLUNG VON ENTZÜNDLICHEN AUTOIMMUNKRANKHEITEN MIT ERZEUGUNG EINER TH1- UND/ODER TH17-REAKTION
28 KDA GST PROTEINS FROM SCHISTOSOMA FOR THE USE THEREOF IN THE TREATMENT OF INFLAMMATORY AUTOIMMUNE DISEASES GENERATING A TH1 AND/OR TH17 RESPONSE

(30) Priorité: 06.02.2012 FR 1251100
(43) Date de publication de la demande: 17.12.2014
(73) Titulaire: Université de Lille, 59800 Lille (FR); INSERM - Institut National de la Santé et de la Recherche Médicale, 75654 Paris Cedex (FR); Centre National de la Recherche Scientifique, 75794 Paris Cedex 16 (FR); Centre Hospitalier Regional Universitaire de Lille, 59037 Lille Cédex (FR)
(72) Inventeur: CAPRON, Monique, F-59133 Phalempin (FR); EL NADY, Mohamed, F-59139 Wattignies (FR); COLOMBEL, Jean-Frédéric, F-59000 Lille (FR); RIVEAU, Gilles, F-59133 Phalempin (FR)
(74) Mandataire: Cabinet Plasseraud
(86) Numéro de dépôt international: PCT/FR2013/050252
(87) Numéro de publication internationale: WO 2013/117860

(56) Documents cités:
- FR-A1- 2 688 008
- LI GUANG-FU ET AL: "Identification of immunodominant Th1-type T cell epitopes from Schistosoma japonicum 28 kDa glutathione-S-transferase, a vaccine candidate", ACTA BIOCHIMICA ET BIOPHYSICA SINICA, vol. 37, no. 11, novembre 2005 (2005-11), pages 751-758, XP002674842, ISSN: 1672-9145
- ALLAMEH A ET AL: "The influence of uric acid treatments on liver glutathione system prevent oxidative damages in experimental autoimmune encephalomyelitis mice", NEUROSCIENCE LETTERS 20080704 IE LNKD- DOI:10.1016/J.NEULET.2008.04.053, vol. 439, no. 1, 4 juillet 2008 (2008-07-04), pages 111-115, XP002674843, ISSN: 0304-3940
- HERVE MAXIME ET AL: "Pivotal roles of the parasite PGD2 synthase and of the host D prostanoid receptor 1 in schistosome immune evasion.", EUROPEAN JOURNAL OF IMMUNOLOGY, vol. 33, no. 10, octobre 2003 (2003-10), pages 2764-2772, XP002674844, ISSN: 0014-2980
- OSADA YOSHIO ET AL: "Parasitic Helminths: New Weapons against Immunological Disorders", JOURNAL OF BIOMEDICINE & BIOTECHNOLOGY, 2010, XP002696665,
- EL-MALKY M ET AL: "Helminth infections: therapeutic potential in autoimmune disorders", PARASITE IMMUNOLOGY (OXFORD), vol. 33, no. 11, novembre 2011 (2011-11), pages 589-593, XP002696666,

### SEQUENCE LISTING

<110> Université LILLE 2
   CAPRON, Monique
<120> Protéine 28 KDa GST provenant de schistosomes pour leur utilisation dans le traitement des maladies inflammatoires auto-immunes engendrant une réponse de type Th1 et/ou Th17
<130> IFB 12
<160> 6
<170> PatentIn version 3.5
<210> 1
   <211> 212
   <212> PRT
   <213> Schistosoma haematobium Sh28GST
<400> 1
   Met Met Thr Gly Asp His Ile Lys Val Ile Tyr Phe Asn Gly Arg Gly
   1               5                   10                  15
   Arg Ala Glu Ser Ile Arg Met Thr Leu Val Ala Ala Gly Val Asn Tyr
               20                  25                  30
   Glu Asp Glu Arg Ile Ser Phe Gln Asp Trp Pro Lys Ile Lys Pro Thr
           35                  40                  45
   Ile Pro Gly Gly Arg Leu Pro Ala Val Lys Ile Thr Asp Asn His Gly
       50                  55                  60
   His Val Lys Trp Met Val Glu Ser Leu Ala Ile Ala Arg Tyr Met Ala
   65                  70                  75                  80
   Lys Lys His His Met Met Gly Gly Thr Glu Glu Glu Tyr Tyr Asn Val
                   85                  90                  95
   Glu Lys Leu Ile Gly Gln Ala Glu Asp Leu Glu His Glu Tyr Tyr Lys
               100                 105                 110
   Thr Leu Met Lys Pro Glu Glu Glu Lys Gln Lys Ile Ile Lys Glu Ile
           115                 120                 125
   Leu Asn Gly Lys Val Pro Val Leu Leu Asp Ile Ile Cys Glu Ser Leu
       130                 135                 140
   Lys Ala Ser Thr Gly Lys Leu Ala Val Gly Asp Lys Val Thr Leu Ala
   145                 150                 155                 160
   Asp Leu Val Leu Ile Ala Val Ile Asp His Val Thr Asp Leu Asp Lys
                   165                 170                 175
   Glu Phe Leu Thr Gly Lys Tyr Pro Glu Ile His Lys His Arg Glu Asn
               180                 185                 190
   Leu Leu Ala Ser Ser Pro Arg Leu Ala Lys Tyr Leu Ser Asp Arg Ala
           195                 200                 205
   Ala Thr Pro Phe
       210
<210> 2
   <211> 211
   <212> PRT
   <213> Schistosoma mansoni Sm28GST
<400> 2
   Met Ala Gly Glu His Ile Lys Val Ile Tyr Phe Asp Gly Arg Gly Arg
   1               5                   10                  15
   Ala Glu Ser Ile Arg Met Thr Leu Val Ala Ala Gly Val Asp Tyr Glu
               20                  25                  30
   Asp Glu Arg Ile Ser Phe Gln Asp Trp Pro Lys Ile Lys Pro Thr Ile
           35                  40                  45
   Pro Gly Gly Arg Leu Pro Ala Val Lys Val Thr Asp Asp His Gly His
       50                  55                  60
   Val Lys Trp Met Leu Glu Ser Leu Ala Ile Ala Arg Tyr Met Ala Lys
   65                  70                  75                  80
   Lys His His Met Met Gly Glu Thr Asp Glu Glu Tyr Tyr Ser Val Glu
                   85                  90                  95
   Lys Leu Ile Gly Gln Ala Glu Asp Val Glu His Glu Tyr His Lys Thr
               100                 105                 110
   Leu Met Lys Pro Gln Glu Glu Lys Glu Lys Ile Thr Lys Glu Ile Leu
           115                 120                 125
   Asn Gly Lys Val Pro Val Leu Leu Asn Met Ile Cys Glu Ser Leu Lys
       130                 135                 140
   Gly Ser Thr Gly Lys Leu Ala Val Gly Asp Lys Val Thr Leu Ala Asp
   145                 150                 155                 160
   Leu Val Leu Ile Ala Val Ile Asp His Val Thr Asp Leu Asp Lys Gly
                   165                 170                 175
   Phe Leu Thr Gly Lys Tyr Pro Glu Ile His Lys His Arg Glu Asn Leu
               180                 185                 190
   Leu Ala Ser Ser Pro Arg Leu Ala Lys Tyr Leu Ser Asn Arg Pro Ala
           195                 200                 205
   Thr Pro Phe
       210
<210> 3
   <211> 211
   <212> PRT
   <213> Schistosoma bovis Sb28GST
<400> 3
   Met Thr Gly Asp His Ile Lys Val Ile Tyr Phe Asn Gly Arg Gly Arg
   1               5                   10                  15
   Ala Glu Ser Ile Arg Met Thr Leu Val Ala Ala Gly Val Asn Tyr Glu
               20                  25                  30
   Asp Glu Arg Ile Ser Phe Gln Asp Trp Pro Lys Ile Lys Pro Thr Ile
           35                  40                  45
   Pro Gly Gly Arg Leu Pro Ala Val Lys Ile Thr Asp Asn His Gly His
       50                  55                  60
   Val Lys Trp Met Leu Glu Ser Leu Ala Ile Ala Arg Tyr Met Ala Lys
   65                  70                  75                  80
   Lys His His Met Met Gly Glu Thr Asp Glu Glu Tyr Tyr Asn Val Glu
                   85                  90                  95
   Lys Leu Ile Gly Gln Val Glu Asp Leu Glu His Glu Tyr His Lys Thr
               100                 105                 110
   Leu Met Lys Pro Glu Glu Glu Lys Gln Lys Ile Thr Lys Glu Ile Leu
           115                 120                 125
   Asn Gly Lys Val Pro Val Leu Leu Asp Ile Ile Cys Glu Ser Leu Lys
       130                 135                 140
   Ala Ser Thr Gly Lys Leu Ala Val Gly Asp Lys Val Thr Leu Ala Asp
   145                 150                 155                 160
   Leu Val Leu Ile Ala Val Ile Asp His Val Thr Asp Leu Asp Lys Glu
                   165                 170                 175
   Phe Leu Thr Gly Lys Tyr Pro Glu Ile His Lys His Arg Glu Asn Leu
               180                 185                 190
   Leu Ala Ser Ser Pro Arg Leu Ala Lys Tyr Leu Ser Asp Arg Ala Ala
           195                 200                 205
   Thr Pro Phe
       210
<210> 4
   <211> 803
   <212> DNA
   <213> Schistosoma haematobium SH28GST
<400> 4
   tctgtctgac tgtatgatga ctggtgatca tatcaaggtt atctatttta acggacgcgg      60
   acgagctgaa tcgatccgga tgacacttgt ggcagctggt gtgaactacg aagatgagag     120
   aattagtttc caagattggc cgaaaatcaa accaactatt ccgggcggac gattgcctgc     180
   agtgaaaatc accgataatc atgggcacgt gaaatggatg gtagagagtt tggctattgc     240
   acggtatatg gcgaagaagc atcatatgat gggaggaaca gaagaggagt attataatgt     300
   tgagaagttg attggtcagg ctgaagatct agaacatgaa tattacaaaa ctttgatgaa     360
   gccagaagaa gagaaacaga agataatcaa agagatactg aacggcaaag taccagttct     420
   tctcgatatt atctgcgaat ctctgaaagc gtccacaggc aagctggctg ttggggataa     480
   agtgactcta gccgacttag ttctgattgc tgtcattgac catgtgactg atctggataa     540
   agaatttcta actggcaagt atcctgagat ccataaacat agagaaaatc tactagccag     600
   ttcaccgaga ttggcgaaat atttatcaga cagggctgca actcccttct agaactgtca     660
   acagaatgct gggtgtgacg agattgaaga tactgatagt agtgcactgg tgcgaccttt     720
   ttactaagac gtcatttgtt ttatggtatt ttttttcgca atcgttatta aaataaactt     780
   agttttctgt ttaaaaaaaa aaa                                             803
<210> 5
   <211> 211
   <212> PRT
   <213> Schistosoma japonicum Sj28GST
<400> 5
   Met Ala Cys Gly His Val Lys Leu Ile Tyr Phe Asn Gly Arg Gly Arg
   1               5                   10                  15
   Ala Glu Pro Ile Arg Met Ile Leu Val Ala Ala Gly Val Glu Phe Glu
               20                  25                  30
   Asp Glu Arg Ile Glu Phe Gln Asp Trp Pro Lys Ile Lys Pro Thr Ile
           35                  40                  45
   Pro Gly Gly Arg Leu Pro Ile Val Lys Ile Thr Asp Lys Arg Gly Asp
       50                  55                  60
   Val Lys Thr Met Ser Glu Ser Leu Ala Ile Ala Arg Phe Ile Ala Arg
   65                  70                  75                  80
   Lys His Asn Met Met Gly Asp Thr Asp Asp Glu Tyr Tyr Ile Ile Glu
                   85                  90                  95
   Lys Met Ile Gly Gln Val Glu Asp Val Glu Ser Glu Tyr His Lys Thr
               100                 105                 110
   Leu Met Lys Pro Pro Glu Glu Lys Glu Lys Ile Ser Lys Glu Ile Leu
           115                 120                 125
   Asn Gly Lys Val Pro Ile Leu Leu Gln Ala Ile Cys Glu Thr Leu Lys
       130                 135                 140
   Glu Ser Thr Gly Asn Leu Thr Val Gly Asp Lys Val Thr Leu Ala Asp
   145                 150                 155                 160
   Val Val Leu Ile Ala Ser Ile Asp His Ile Thr Asp Leu Asp Lys Glu
                   165                 170                 175
   Phe Leu Thr Gly Lys Tyr Pro Glu Ile His Lys His Arg Lys His Leu
               180                 185                 190
   Leu Ala Thr Ser Pro Lys Leu Ala Lys Tyr Leu Ser Glu Arg His Ala
           195                 200                 205
   Thr Ala Phe
       210
<210> 6
   <211> 218
   <212> PRT
   <213> Schistosoma japonicum Sj26GST
<400> 6
   Met Ser Pro Ile Leu Gly Tyr Trp Lys Ile Lys Gly Leu Val Gln Pro
   1               5                   10                  15
   Thr Arg Leu Leu Leu Glu Tyr Leu Glu Glu Lys Tyr Glu Glu His Leu
               20                  25                  30
   Tyr Glu Arg Asp Glu Gly Asp Lys Trp Arg Asn Lys Lys Phe Glu Leu
           35                  40                  45
   Gly Leu Glu Phe Pro Asn Leu Pro Tyr Tyr Ile Asp Gly Asp Val Lys
       50                  55                  60
   Leu Thr Gln Ser Met Ala Ile Ile Arg Tyr Ile Ala Asp Lys His Asn
   65                  70                  75                  80
   Met Leu Gly Gly Cys Pro Lys Glu Arg Ala Glu Ile Ser Met Leu Glu
                   85                  90                  95
   Gly Ala Val Leu Asp Ile Arg Tyr Gly Val Ser Arg Ile Ala Tyr Ser
               100                 105                 110
   Lys Asp Phe Glu Thr Leu Lys Val Asp Phe Leu Ser Lys Leu Pro Glu
           115                 120                 125
   Met Leu Lys Met Phe Glu Asp Arg Leu Cys His Lys Thr Tyr Leu Asn
       130                 135                 140
   Gly Asp His Val Thr His Pro Asp Phe Met Leu Tyr Asp Ala Leu Asp
   145                 150                 155                 160
   Val Val Leu Tyr Met Asp Pro Met Cys Leu Asp Ala Phe Pro Lys Leu
                   165                 170                 175
   Val Cys Phe Lys Lys Arg Ile Glu Ala Ile Pro Gln Ile Asp Lys Tyr
               180                 185                 190
   Leu Lys Ser Ser Lys Tyr Ile Ala Trp Pro Leu Gln Gly Trp Gln Ala
           195                 200                 205
   Thr Phe Gly Gly Gly Asp His Pro Pro Lys
       210                 215

## Description

La présente invention concerne des protéines particulières qui sont des glutathion-S-transférases provenant de différents parasites schistosomes, pour leur utilisation dans le traitement de maladies inflammatoires auto-immunes telles que la maladie de Crohn ou la sclérose en plaques. La présente invention concerne également les fragments desdites protéines, les séquences dérivées de ces protéines et homologues à ces protéines pour autant que ces séquences et ces fragments puissent engendrer une réponse anti-inflammatoire.

La présente invention concerne également une composition pharmaceutique comprenant les objets précités et au moins un excipient pharmacologiquement acceptable.

Dans les pays développés, les maladies inflammatoires chroniques liées à un dérèglement du système immunitaire (maladies inflammatoires auto-immunes) touchent environ 8% de la population. Parmi les maladies inflammatoires auto-immunes les plus répandues, on peut citer, les maladies inflammatoires chroniques de l'intestin (MICI) et la sclérose en plaques.

Les maladies inflammatoires chroniques de l'intestin (maladie de Crohn et rectocolite hémorragique) sont de plus en plus fréquentes dans les pays dits « développés ». Récemment, on assiste malheureusement, dans ces pays, à une augmentation des cas pédiatriques de ces pathologies. Par ailleurs, ces maladies, commencent à faire leur apparition dans les pays dits « en voie de développement », du fait probablement du changement des habitudes alimentaires et des conditions sanitaires. Les traitements classiques des maladies inflammatoires chroniques auto-immunes sont à base d'immunosuppresseurs et/ou de corticostéroïdes. Ces traitements ne sont pas dénués d'effets secondaires graves.

Ainsi, pour ce qui est de la maladie de Crohn, l'azathioprine, le méthotrexate ou la cyclosporine, qui sont des immunosuppresseurs, présentent une efficacité variable et peuvent être associés à des effets secondaires graves comme des syndromes lympho-prolifératifs ou des fibroses hépatiques.

Les formes aiguës de la maladie de Crohn sont traitées avec des corticostéroïdes dont la toxicité est élevée et qui favorisent l'apparition de l'ostéoporose, du diabète et des infections opportunistes.

Les thérapies biologiques mettent en oeuvre l'inhibition du TNF via des anticorps monoclonaux anti-TNF. Ces thérapies permettent de réduire les hospitalisations et les interventions chirurgicales mais engendrent des risques d'infection, de lymphome ou de défaillance cardiaque. De plus, certains patients ne sont pas réactifs à ce type de thérapie qui reste, de plus, très coûteuse. Par ailleurs, chaque récidive de la maladie risque d'engendrer des conséquences invalidantes pour le patient, comme par exemple, la pose d'une poche de récupération des fèces. La qualité de vie du patient est grandement diminuée.

Dans les formes pédiatriques de ces maladies, et en particulier dans le cas de la maladie de Crohn dont l'incidence a beaucoup augmenté lors des dix dernières années, l'arsenal thérapeutique est très limité. Les immunosuppresseurs ne peuvent pas être utilisés.

Il y a donc un besoin réel et urgent de mettre au point un nouveau traitement préventif et/ou curatif des maladies inflammatoires chroniques liées à un dérèglement du système immunitaire engendrant une réponse inflammatoire de type Th1 et/ou Th17, comme la maladie de Crohn ou la sclérose en plaques, par exemple.

Un premier but de la présente invention est de proposer un nouveau principe actif pour son utilisation dans le traitement préventif et/ou curatif des maladies inflammatoires chroniques engendrant une réponse inflammatoire de type Th1 et/ou Th17.

Un autre but de la présente invention est de proposer un principe actif pour son utilisation dans le traitement préventif et/ou curatif des maladies précitées qui présente une faible toxicité et peu ou pas d'effets secondaires.

Un autre but de la présente invention est de proposer un principe actif pour son utilisation dans le traitement des maladies précitées qui soit susceptible d'être administré aux enfants.

Au moins un de ces buts est atteint au moyen d'un produit choisi parmi une protéine, un fragment de ladite protéine, une séquence dérivée et une séquence homologue de ladite protéine. La présente demande décrit, de manière caractéristique, que
- ladite protéine comprend ou est constituée par la protéine 28KDa Glutathion S-transférase d'un schistosome choisi parmi *Schistosoma haematobium, Schistosoma mansoni, Schistosoma bovis* représentée respectivement par les séquences SEQ ID NO : 1, SEQ ID NO : 2, et SEQ ID NO : 3 ;
- ledit fragment est un fragment d'une desdites protéines sous réserve qu'il induise une réponse immunitaire de type Th2, ledit fragment comportant, de préférence, au moins la région comprise de l'acide aminé en position 15 à l'acide aminé en position 60, notamment la région comprise de l'acide aminé en position 20 à l'acide aminé en position 50, de préférence la région comprise de l'acide aminé en position 24 à l'acide aminé en position 43 et la région comprise de l'acide aminé en position 170 à l'acide aminé en position 220, notamment la région comprise de l'acide aminé en position 180 à l'acide aminé en position 215, de préférence la région comprise de l'acide aminé en position 190 à l'acide aminé en position 211 de la séquence SEQ ID NO : 1, SEQ ID NO : 2 ou SEQ ID NO : 3 ;
- ladite séquence dérivée est dérivée d'une desdites séquences SEQ ID NO : 1, SEQ ID NO : 2 et SEQ ID NO : 3 ou desdits fragments précités et obtenue notamment par suppression, substitution ou ajout d'un ou plusieurs acides aminés, sous réserve que cette séquence dérivée induise une réponse de type Th2 ;
- ladite séquence homologue est une séquence homologue d'une desdites séquence SEQ ID NO : 1, SEQ ID NO : 2 et SEQ ID NO : 3 ou d'un desdits fragments, et présente notamment une identité d'au moins 80% et notamment d'au moins 85% avec une desdites séquences SEQ ID NO : 1, SEQ ID NO : 2 et SEQ ID NO : 3, sous réserve que ladite séquence homologue induise une réponse de type Th2 ;
pour son utilisation dans le traitement préventif et/ou curatif d'une maladie inflammatoire chronique liée à un dérèglement du système immunitaire générant une réponse immunitaire de type Th1/Th17.

Selon un mode particulier, l'invention concerne un produit choisi parmi une protéine, un fragment de ladite protéine, une séquence dérivée et une séquence homologue de ladite protéine, et de manière caractéristique :
- ladite protéine comprend ou est constituée par la protéine 28KDa Glutathion S-transférase d'un schistosome choisi parmi *Schistosoma haematobium, Schistosoma mansoni, Schistosoma bovis* représentée respectivement par les séquences SEQ ID NO : 1, SEQ ID NO : 2, et SEQ ID NO : 3 ;
- ledit fragment est un fragment d'une desdites protéines comportant au moins une région comprise parmi les régions suivantes : la région comprise de l'acide aminé en position 15 à l'acide aminé en position 60, la région comprise de l'acide aminé en position 20 à l'acide aminé en position 50, la région comprise de l'acide aminé en position 24 à l'acide aminé en position 43, la région comprise de l'acide aminé en position 170 à l'acide aminé en position 220, notamment la région comprise de l'acide aminé en position 180 à l'acide aminé en position 215, de préférence la région comprise de l'acide aminé en position 190 à l'acide aminé en position 211 de la séquence SEQ ID NO : 1, SEQ ID NO : 2 ou SEQ ID NO : 3 ; ceci induisant une réponse immunitaire de type Th2, caractérisée par la production d'IL-4, d'IL-5 ou d'IL-13 par des lymphocytes ne produisant pas d'IFN gamma ;
et ceci diminuant la réponse immunitaire de type Th1, la réponse immunitaire de type Th1 étant caractérisée par la production d'IFN gamma et de TNF par des lymphocytes ne produisant pas d'IL-4 ou d'IL-5, et la production d'IL-1b par les tissus coliques de rat ;
- ladite séquence dérivée est dérivée d'une desdites séquences SEQ ID NO : 1, SEQ ID NO : 2 et SEQ ID NO : 3 ou d'un desdits fragments précités et obtenue notamment par suppression, substitution ou ajout d'un ou plusieurs acides aminés, dans la séquence SEQ ID NO : 1, SEQ ID NO : 2 ou SEQ ID NO : 3, ladite séquence dérivée comprenant au moins une région comprise parmi les régions suivantes : la région comprise de l'acide aminé en position 15 à l'acide aminé en position 60, la région comprise de l'acide aminé en position 20 à l'acide aminé en position 50, la région comprise de l'acide aminé en position 24 à l'acide aminé en position 43, la région comprise de l'acide aminé en position 170 à l'acide aminé en position 220, la région comprise de l'acide aminé en position 180 à l'acide aminé en position 215 et la région comprise de l'acide aminé en position 190 à l'acide aminé en position 211 de la séquence SEQ ID NO : 1, SEQ ID NO : 2 ou SEQ ID NO : 3 ; ceci entraînant l'induction d'une réponse Th2 et/ou la diminution de la réponse Th1, caractérisées respectivement par la production d'IL-4, d'IL-5 ou d'IL-13, et la production d'IFN gamma, de TNF ou d'IL-1b ; et
- ladite séquence homologue est une séquence homologue d'une desdites séquence SEQ ID NO : 1, SEQ ID NO : 2 et SEQ ID NO : 3 ou d'un desdits fragments, et présente une identité d'au moins 80% ou d'au moins 85% avec au moins l'une des régions suivantes desdites séquences : la région comprise de l'acide aminé en position 15 à l'acide aminé en position 60, la région comprise de l'acide aminé en position 20 à l'acide aminé en position 50, la région comprise de l'acide aminé en position 24 à l'acide aminé en position 43, la région comprise de l'acide aminé en position 170 à l'acide aminé en position 220, la région comprise de l'acide aminé en position 180 à l'acide aminé en position 215, et la région comprise de l'acide aminé en position 190 à l'acide aminé en position 211 de la séquence SEQ ID NO : 1, SEQ ID NO : 2 ou SEQ ID NO : 3 ; ou avec la totalité de l'une des séquences SEQ ID NO : 1, SEQ ID NO : 2 ou SEQ ID NO : 3 ; ceci entraînant l'induction d'une réponse Th2 et/ou la diminution de la réponse Th1, caractérisées respectivement par la production d'IL-4, d'IL-5 ou d'IL-13, et la production d'IFN gamma, de TNF ou d'IL-1b ;
pour son utilisation dans le traitement préventif et/ou curatif d'une maladie inflammatoire chronique liée à un dérèglement du système immunitaire générant une réponse immunitaire de type Th1/Th17 choisie parmi parmi les maladies inflammatoires auto-immunes suivantes : la maladie de Berger, la maladie de Basedow, la thyroïdite d'Hashimoto, le myxoedème primaire, la maladie coeliaque, la rectocolite hémorragique, la maladie de Crohn, l'arthrite rhumatoïde, la cirrhose biliaire primitive, la cholangite sclérosante primitive, les anémies hémolytiques auto-immunes, l'anémie de Biermer (Anémie pernicieuse), le lupus érythémateux, le syndrome de CREST, le diabète de type 1, la sclérodermie, le pemphigus vulgaire, la pemphigoïde bulleuse, l'épidermolyse Bulleuse Acquise, la dermatite herpétiforme, la myasthénie, le syndrome myasthénique de Lambert-Eaton, la polymyosite, le syndrome de Goujerot-Sjôgren, la sclérose en plaques, la maladie de Graves et le psoriasis.

La présente demande concerne également un produit choisi parmi une protéine, un fragment de ladite protéine, une séquence dérivée et une séquence homologue de ladite protéine, et de manière caractéristique :
- ladite protéine comprend ou est constituée par la protéine 28KDa Glutathion S-transférase d'un schistosome choisi parmi *Schistosoma haematobium, Schistosoma mansoni, Schistosoma bovis* représentée respectivement par les séquences SEQ ID NO : 1, SEQ ID NO : 2, et SEQ ID NO : 3 ;
- ledit fragment est un fragment d'une desdites protéines sous réserve qu'il induise une réponse immunitaire de type Th2 et/ou diminue la réponse de type Th1, ledit fragment comportant, au moins une région comprise parmi les régions suivantes : la région comprise de l'acide aminé en position 15 à l'acide aminé en position 60, la région comprise de l'acide aminé en position 20 à l'acide aminé en position 50, la région comprise de l'acide aminé en position 24 à l'acide aminé en position 43, la région comprise de l'acide aminé en position 170 à l'acide aminé en position 220, notamment la région comprise de l'acide aminé en position 180 à l'acide aminé en position 215, de préférence la région comprise de l'acide aminé en position 190 à l'acide aminé en position 211 de la séquence SEQ ID NO : 1, SEQ ID NO : 2 ou SEQ ID NO : 3 ;
- ladite séquence dérivée est dérivée d'une desdites séquences SEQ ID NO : 1, SEQ ID NO : 2 et SEQ ID NO : 3 ou d'un desdits fragments précités et obtenue notamment par suppression, substitution ou ajout d'un ou plusieurs acides aminés, dans la séquence SEQ ID NO : 1, SEQ ID NO : 2 ou SEQ ID NO : 3, ladite séquence dérivée comprenant au moins une région comprise parmi les régions suivantes : la région comprise de l'acide aminé en position 15 à l'acide aminé en position 60, la région comprise de l'acide aminé en position 20 à l'acide aminé en position 50, la région comprise de l'acide aminé en position 24 à l'acide aminé en position 43, la région comprise de l'acide aminé en position 170 à l'acide aminé en position 220, la région comprise de l'acide aminé en position 180 à l'acide aminé en position 215 et la région comprise de l'acide aminé en position 190 à l'acide aminé en position 211 de la séquence SEQ ID NO : 1, SEQ ID NO : 2 ou SEQ ID NO : 3 ; ceci entraînant l'induction d'une réponse Th2 et/ou la diminution de la réponse Th1 ; et

- ladite séquence homologue est une séquence homologue d'une desdites séquence SEQ ID NO : 1, SEQ ID NO : 2 et SEQ ID NO : 3 ou d'un desdits fragments, et présente une identité d'au moins 80% et notamment d'au moins 85% avec au moins l'une des régions suivantes desdites séquences : la région comprise de l'acide aminé en position 15 à l'acide aminé en position 60, la région comprise de l'acide aminé en position 20 à l'acide aminé en position 50, la région comprise de l'acide aminé en position 24 à l'acide aminé en position 43, la région comprise de l'acide aminé en position 170 à l'acide aminé en position 220, la région comprise de l'acide aminé en position 180 à l'acide aminé en position 215, et la région comprise de l'acide aminé en position 190 à l'acide aminé en position 211 de la séquence SEQ ID NO : 1, SEQ ID NO : 2 ou SEQ ID NO : 3 ; ou avec la totalité de l'une des séquences SEQ ID NO : 1, SEQ ID NO : 2 ou SEQ ID NO : 3 ; ceci entraînant l'induction d'une réponse Th2 et/ou la diminution de la réponse Th1.
pour son utilisation dans le traitement préventif et/ou curatif d'une maladie inflammatoire chronique liée à un dérèglement du système immunitaire générant une réponse immunitaire de type Th1/Th17.

Selon l'invention le fragment est un fragment d'une desdites protéines et comporte de préférence, au moins la région comprise de l'acide aminé en position 15 à l'acide aminé en position 60, notamment la région comprise de l'acide aminé en position 20 à l'acide aminé en position 50, de préférence la région comprise de l'acide aminé en position 24 à l'acide aminé en position 43 et la région comprise de l'acide aminé en position 170 à l'acide aminé en position 220, notamment la région comprise de l'acide aminé en position 180 à l'acide aminé en position 215, de préférence la région comprise de l'acide aminé en position 190 à l'acide aminé en position 211 de la séquence SEQ ID NO : 1, SEQ ID NO : 2 ou SEQ ID NO : 3,
ceci induisant une réponse immunitaire de type Th2, caractérisée par la production d'IL-4, d'IL-5 ou d'IL-13 par des lymphocytes ne produisant pas d'IFN gamma ;
et ceci diminuant la réponse immunitaire de type Th1, la réponse immunitaire de type Th1 étant caractérisée par la production d'IFN gamma et de TNF par des lymphocytes ne produisant pas d'IL-4 ou d'IL-5, et la production d'IL-1b par les tissus coliques de rat ;
Lorsque le produit de l'invention est une séquence dérivée d'une desdites séquences SEQ ID NO : 1, SEQ ID NO : 2 et SEQ ID NO : 3, cette dernière peut avantageusement comporter au moins la région comprise de l'acide aminé en position 15 à l'acide aminé en position 60, notamment la région comprise de l'acide aminé en position 20 à l'acide aminé en position 50, de préférence la région comprise de l'acide aminé en position 24 à l'acide aminé en position 43 et la région comprise de l'acide aminé en position 170 à l'acide aminé en position 220, notamment la région comprise de l'acide aminé en position 180 à l'acide aminé en position 215, de préférence, la région comprise de l'acide aminé en position 190 à l'acide aminé en position 211 de la séquence SEQ ID NO : 1, SEQ ID NO : 2 ou SEQ ID NO : 3, ceci induisant une réponse Th2 et/ou la diminution de la réponse Th1.

Lorsque le produit de l'invention est une séquence homologue telle que précitée, elle comporte avantageusement au moins la région comprise de l'acide aminé en position 15 à l'acide aminé en position 60, notamment la région comprise de l'acide aminé en position 20 à l'acide aminé en position 50, de préférence la région comprise de l'acide aminé en position 24 à l'acide aminé en position 43 et la région comprise de l'acide aminé en position 170 à l'acide aminé en position 220, notamment la région comprise de l'acide aminé en position 180 à l'acide aminé en position 215, de préférence la région comprise de l'acide aminé en position 190 à l'acide aminé en position 211 de la séquence SEQ ID NO : 1, SEQ ID NO : 2 ou SEQ ID NO : 3, ceci induisant une réponse Th2 et/ou la diminution de la réponse Th1.

La séquence homologue peut donc comprendre au moins une des régions précitées ou toutes les régions précitées. Ces régions pouvant être identiques aux régions correspondantes des séquences SEQ ID NO 1, SEQ ID NO 2 et SEQ ID NO 3 ou présenter chacune une homologie d'au moins 80% et de préférence d'au moins 85% avec la région correspondante d'une des séquences précitées. Le pourcentage d'homologie peut être différent pour chaque région.

On définit, selon la présente invention une réponse de type Th1 comme étant caractérisée par la présence de lymphocytes produisant des cytokines comme l'IFN gamma mais ne produisant pas d'IL-4 ou d'IL-5. Cette réponse conduit également à la production de TNF.

Une réponse de type Th2 est caractérisée par la présence de lymphocytes produisant des cytokines comme l'IL-4, l'IL-5 ou l'IL-13 mais sans production d"IFN gamma.

La réponse de type Th17 est une réponse inflammatoire où les lymphocytes produisent de l'IL-17 ou de l'IL-25, en plus du TNF produit par de nombreux types cellulaires.

On mesure donc le type de réponse en évaluant les cytokines produites soit par les cellules elles-mêmes (par cytométrie en flux) quand on a accès aux cellules, soit par PCR dans les tissus.On parle de « type » de réponse ; le type de réponse correspond à la réponse majoritaire des cellules ou de l'organisme. En pratique, il y a toujours des réponses mixtes. Un moyen de mesurer la réponse Th1 versus Th2 est de mesurer le rapport IFNgamma / IL-4.

Les pathologies auto-immunes génèrent souvent des réponses mixtes et surtout pro-inflammatoires, d'où l'utilisation des biothérapies à base d'anti-TNF.

Selon un mode de réalisation, ladite protéine est représentée par la séquence SEQ ID NO : 1 (28 GST glutathion transférase de *Schistosoma haematobium*), ledit fragment est un fragment de ladite protéine comprenant notamment la région comprise de l'acide aminé en position 15 à l'acide aminé en position 60, notamment la région comprise de l'acide aminé en position 20 à l'acide aminé en position 50, de préférence la région comprise de l'acide aminé en position 24 à l'acide aminé en position 43 et la région comprise de l'acide aminé en position 170 à l'acide aminé en position 220, notamment la région comprise de l'acide aminé en position 180 à l'acide aminé en position 215, de préférence la région comprise de l'acide aminé en position 190 à l'acide aminé en position 211, ladite séquence dérivée est obtenue à partir de SEQ ID NO : 1 et ladite séquence homologue présente une identité telle que précitée avec la séquence SEQ ID NO : 1.

Selon un autre mode de réalisation particulier, ledit produit est le produit d'expression d'une séquence nucléotidique codant pour :
- la protéine 28KDa Glutathion-S-transferase représentée par SEQ ID NO : 1 ; ou
- un fragment de ladite protéine représentée par SEQ ID NO : 1, ledit fragment comportant ou étant constitué d'au moins la région comprise de l'acide aminé en position 15 à l'acide aminé en position 60, notamment la région comprise de l'acide aminé en position 20 à l'acide aminé en position 50, de préférence la région comprise de l'acide aminé en position 24 à l'acide aminé en position 43 et la région comprise de l'acide aminé en position 170 à l'acide aminé en position 220, notamment la région comprise de l'acide aminé en position 180 à l'acide aminé en position 215, de préférence la région comprise de l'acide aminé en position 190 à l'acide aminé en position 211 de la séquence SEQ ID NO : 1 ; ou
- une séquence dérivée telle que précitée ; ou
- une séquence homologue telle que précitée ;
ladite séquence nucléotidique codante correspondant notamment à la séquence SEQ ID NO : 4.

Selon un mode réalisation particulier, la protéine est le produit d'expression de ladite séquence codante SEQ ID NO : 4 dans *Saccharomyces cerevisiae* ou dans *Escherichia coli.*

Selon l'invention, la maladie précédemment définie est choisie parmi les maladies inflammatoires auto-immunes suivantes : la maladie de Berger, la maladie de Basedow, la thyroïdite d'Hashimoto, le myxoedème primaire, la maladie coeliaque, la rectocolite hémorragique, la maladie de Crohn, l'arthrite rhumatoïde, la cirrhose biliaire primitive, la cholangite sclérosante primitive, les anémies hémolytiques auto-immunes, l'anémie de Biermer (Anémie pernicieuse), le lupus érythémateux, le syndrome de CREST, le diabète de type 1, la sclérodermie, le pemphigus vulgaire, la pemphigoïde bulleuse, l'épidermolyse Bulleuse Acquise, la dermatite herpétiforme, la myasthénie, le syndrome myasthénique de Lambert-Eaton, la polymyosite, le syndrome de Goujerot-Sjôgren, la sclérose en plaques, la maladie de Graves et le psoriasis.

Selon l'invention, le traitement de la pathologie précitée peut s'appliquer à tout patient, humain ou animal. Il s'applique en particulier à l'être humain, adulte ou enfant. Selon l'invention, le traitement peut concerner plus particulièrement un enfant et plus particulièrement le traitement préventif chez l'enfant.

On définit par « enfant », un individu âgé de 0 à 18 ans, plus particulièrement âgé de 9 à 18 ans.

La présente invention concerne également une composition pharmaceutique, ladite composition comprenant, en tant qu'agent actif, un produit selon l'invention et un excipient pharmacologiquement acceptable, notamment pour le traitement préventif et/ou curatif d'une maladie inflammatoire chronique liée à un dérèglement du système auto-immunitaire générant une réponse immunitaire de type Th1 et/ou Th17.

Par « pharmacologiquement acceptable », on entend au sens de la présente invention, tout excipient susceptible d'être injecté, ingéré ou au moins appliqué sur la peau sans engendrer de réaction secondaire qui serait, de manière statistique, supérieure au bénéfice procuré par le traitement.

Selon l'invention, la composition est avantageusement utilisée dans le traitement préventif et/ou curatif d'une maladie choisie parmi les maladies inflammatoires auto-immunes suivantes : la maladie de Berger, la maladie de Basedow, la thyroïdite d'Hashimoto, le myxoedème primaire, la maladie coeliaque, la rectocolite hémorragique, la maladie de Crohn, l'arthrite rhumatoïde, la cirrhose biliaire primitive, la cholangite sclérosante primitive, les anémies hémolytiques auto-immunes, l'anémie de Biermer (Anémie pernicieuse), le lupus érythémateux, le syndrome de CREST, le diabète de type 1, la sclérodermie, le pemphigus vulgaire, la pemphigoïde bulleuse, l'épidermolyse Bulleuse Acquise, la dermatite herpétiforme, la myasthénie, le syndrome myasthénique de Lambert-Eaton, la polymyosite, le syndrome de Goujerot-Sjôgren, la sclérose en plaques, la polyarthrite rhumatoïde, la maladie de Graves et le psoriasis, en particulier les formes pédiatriques de ces pathologies, lorsqu'elles existent, et notamment le traitement à titre préventif et plus particulièrement le traitement préventif des formes pédiatriques.

L'action immunogène du produit et/ou de la composition selon l'invention peut être la réduction ou la suppression de la réaction inflammatoire (de type Th1 notamment) et/ou l'induction d'une réponse immune de type Th2 via, par exemple, la sécrétion de certaines interleukines.

La présente invention concerne également ladite composition pharmaceutique en tant que vaccin et utilisable pour le traitement d'une maladie auto-immune engendrant une réponse de type Th1 et/ou Th17 et en particulier pour le traitement des pathologies précitées. Ce vaccin peut s'adresser notamment aux enfants.

Selon l'invention, la composition peut comprendre un adjuvant, notamment un adjuvant choisi parmi les sels d'aluminium et plus particulièrement l'hydroxyde d'aluminium. L'hydroxyde d'aluminium est un adjuvant de la réponse immunitaire utilisé en vaccinologie et compatible avec l'usage humain. Formant un réseau ionique, les protéines antigéniques s'y adsorbent après quelques minutes d'incubation. Ce complexe crée un dépôt transitoire permettant un recrutement cellulaire et favorisant la réponse antigénique de type Th2.

Le terme « sels d'aluminium » désigne tous les sels existants à l'état naturel ou non de l'aluminium. Il s'agit, par exemple, du sulfate d'aluminium (hydraté ou non), du phosphate d'aluminium, de l'alun (KAl(SO₄)₂.12H₂O), de l'hydroxyde d'aluminium ou de tout autre sel de formule (BAl(SO₄)₂.12H₂O).

Selon l'invention, la concentration en adjuvant est comprise de 0,5 mg/ml à 2 mg/ml, notamment de 0,3 mg/ml à 1 mg/ml et en particulier de 220 µg/ml à 280 µg/ml et de préférence sensiblement égale à 250 µg/ml.

Comme illustré ci-après, de bons résultats sont obtenus avec l'hydroxyde d'aluminium à une concentration comprise dans la plage de concentrations précitée et notamment de 250 µg/ml.

La présente demande divulgue également un produit choisi parmi une protéine, un fragment de ladite protéine, une séquence dérivée et une séquence homologue de ladite protéine, caractérisé en ce que :
- ladite protéine comprend ou est constituée par la protéine 28KDa Glutathion S-transférase de *Schistosoma japonicum ou par la protéine* 26KDa Glutathion S-transférase de *Schistosoma japonicum* représentées respectivement par les séquences SEQ ID NO : 5, et SEQ ID NO : 6,
- en ce que ledit fragment est un fragment d'une desdites protéines sous réserve qu'il induise principalement une réponse immunitaire de type Th2,
- en ce que ladite séquence dérivée est dérivée d'une desdites séquences SEQ ID NO : 5, et SEQ ID NO : 6 ou desdits fragments précités et obtenue notamment par suppression, substitution ou ajout d'un ou plusieurs acides aminés, sous réserve que cette séquence dérivée induise principalement une réponse de type Th2.
- et en ce que ladite séquence homologue est une séquence homologue d'une desdites séquence SEQ ID NO : 5, et SEQ ID NO : 6 ou d'un desdits fragments, et présente notamment une identité d'au moins 80% et notamment d'au moins 85% avec une desdites séquences SEQ ID NO : 5, et SEQ ID NO : 6, sous réserve que ladite séquence homologue induise principalement une réponse de type Th2 ; pour son utilisation dans le traitement préventif et/ou curatif d'une maladie inflammatoire chronique liée à un dérèglement du système immunitaire générant une réponse immunitaire de type Th1 et/ou Th17, en particulier chez un enfant.

La maladie inflammatoire chronique liée à un dérèglement du système immunitaire générant une réponse immunitaire de type Th1 et/ou Th17 peut être choisie parmi la maladie de Crohn, la rectocolite hémorragique, la sclérose en plaques, la polyarthrite rhumatoïde, le diabète de type1 (auto-immun), la maladie de Graves et le psoriasis

La présente invention concerne également une composition pharmaceutique comprenant en tant que qu'ingrédient actif, au moins un des produits tels que précités en référence à Sj28GST et Sj26GST et un excipient pharmacologiquement acceptable.

Cette composition peut également comporter un adjuvant tel que précité et en particulier l'hydroxyde d'aluminium. La quantité d'adjuvant peut être telle que précitée.

La protéine native 28kDa glutathion S-transférase native est une protéine exprimée par les parasites schistosomes, vers plats, responsables de la bilharziose. Il existe plusieurs espèces de schistosomes. *Schistosoma mansoni* est responsable chez l'Homme, de bilharziose intestinale, en Afrique et au Brésil. *Schistosoma haematobium* est responsable, chez l'Homme, de bilharziose urinaire en Afrique et Péninsule arabique. Chaque espèce de schistosome exprime une 28kDa glutathion S-transférase qui lui est propre. Ainsi, l'espèce *Schistosoma mansoni* exprime la Sm28GST, l'espèce *Schistosoma haematobium* exprime la Sh28GST, *Schistosoma bovis* (schistosome infectant le bétail) exprime la Sb28GST et l'espèce *Schistosoma japonicum* (touchant l'Asie du sud-est -Philippines et sud Chine-) exprime la Sj28GST et la Sj26GST. Les gènes codant ces protéines sont connus et/ou l'Homme du Métier est capable de les identifier. L'Homme du Métier est donc à même de produire de manière recombinante les protéines précitées et les produits de l'invention.

Les protéines Sh28GST, Sm28GST, Sb28GST, Sj28GST et Sj26GST ont des séquences qui sont identifiées et répertoriées dans les bases de données, notamment, sur le site http://www.uniprot.org/uniprot/P30114. Les protéines Sb28GST et Sh28GST sont identiques à 97% alors que les protéines Sh28GST et Sm28GST sont identiques à 91%.

Les séquences SEQ ID NO : 1, SEQ ID NO : 2, SEQ ID NO : 3, SEQ ID NO : 5 et SEQ ID NO : 6 ci-après représentent respectivement les séquences des protéines Sh28GST, Sm28GST, Sb28GST, Sj28 GST et Sj26GST.

Il est connu que les parasites induisent une réponse immunitaire de la part de l'hôte infesté. Ainsi, il est connu que le schistosome a une action sur le système immunitaire de l'hôte qu'il infeste de manière à ce que ce dernier ne le rejette pas. La réaction immunitaire de l'hôte commence par une réaction de type Th1, de courte durée puis se poursuit par une réaction de type Th2 et enfin par une réaction T régulatrice. Lorsque se produit la réponse T régulatrice, l'hôte infecté et le parasite coexistent.

Il est connu que les maladies inflammatoires chroniques liées à un dérèglement du système immunitaire engendrent chez les patients, une réponse immunitaire anormale vers les auto-antigènes, ce qui induit une inflammation. Dans les cas de la maladie de Crohn et de la rectocolite hémorragique, on observe une inflammation de la muqueuse intestinale caractérisée par des ulcérations et une infiltration des cellules inflammatoires recrutées par des cytokines et chimio-attractants de type Th1, entre autres.

Dans le cas de la sclérose en plaques, on observe une inflammation locale de la gaine de myéline des axones du cerveau et de la moelle épinière qui engendre une démyélinisation des fibres nerveuses.

Les inventeurs ont montré que, de manière surprenante, la protéine Sh28GSTpermet, en engendrant une réponse anti-inflammatoire de type Th2, de réduire la réponse de type Th1. Cette diminution de la réponse de type Th1 permet de réduire les symptômes liés à la maladie précitée, en particulier l'inflammation. La protéine agissant, notamment sur les interleukines qui sont présentes dans tout l'organisme, elle permet donc de traiter, de manière préventive ou curative, une maladie telle que précitée affectant n'importe quel organe ou même l'organisme entier. Les mêmes effets peuvent être envisagés pour tous les produits de l'invention.

De plus, il est connu qu'il existe un lien entre inflammation et cancer, la première favorisant l'apparition du second. La protéine selon l'invention peut ainsi en réduisant directement ou indirectement l'inflammation chronique, réduire voir éliminer les cancers induits par l'inflammation chronique liée à la maladie inflammatoire auto-immune. Ainsi, la rectocolite hémorragique et la maladie de Crohn sont des facteurs de sur risque du cancer colorectal.

Les résultats ci-après confirment l'effet de la protéine Sh28GST dans le traitement (notamment préventif) de la maladie de Crohn qui est une maladie inflammatoire auto-immune engendrant une réponse de type Th1.

La protéine selon l'invention, quel que soit le schistosome dont elle provient et quel que soit son poids moléculaire peut être naturelle isolée (native) ou recombinante.

Dans toute la présente demande de brevet, les termes de « 28KDa glutathion S-transférase recombinante » (rSh28GST) désignent toute protéine obtenue de manière recombinante par insertion dans un organisme hôte de la séquence totale codant pour la Sh28GST (SEQ NO : 4) ou d'une fraction de cette séquence. Cette synthèse peut être effectuée dans différentes cellules hôtes, bactéries, levures ou cellules supérieures, en fonction du vecteur dans lequel la séquence codante est insérée et des signaux de contrôle d'expression. La protéine recombinante selon la présente invention peut présenter une structure primaire identique à celle de la protéine Sh28GST native, présente chez *Schistosoma haematobium,* mais elle peut aussi être un dérivé de celle-ci ou une protéine Sh28GST incomplète (fragment de protéine) mais ayant une activité immunogène. Cette protéine ou ce fragment de protéine peut également être fusionné à une autre protéine (ou fragment de protéine), suite à une manipulation génétique des segments d'ADN correspondants, afin de favoriser une meilleure expression de la protéine dans la cellule hôte ou éventuellement de provoquer son excrétion hors de la cellule.

Dans les exemples, le terme de rSh28GST désigne la protéine produite dans *Saccharomyces cerevisiae* par l'insertion de la séquence nucléotidique codante SEQ NO : 4.

La protéine rSh28GST est bien connue. Ainsi, l'article «Crystal structure of the 28KDa glutathione S-transferase from *Schistosoma haematobium»* précité cite une méthode de production de la Sh28GST recombinante selon l'invention dans *Escherichia coli.* De même, l'article « Vaccine potential of a recombinant glutathione S-transferase cloned from Schistosoma haematobium in primates experimentally infected with an homologous challenge » publié dans la revue Vaccine en 1999, cite une protéine Sh28GST recombinante produite dans une souche spécifique de *Saccharomyces cerevisiae.*

La protéine recombinante de l'invention n'est néanmoins pas limitée à ces deux exemples précités, les technologies permettant le clonage et l'expression d'un gène étranger ou d'un fragment de gène étranger, dans différentes cellules hôtes étant connues de l'Homme du Métier.

La production de la protéine recombinante dans ces bactéries est déjà connue et parfaitement maîtrisée. En effet, la protéine rSh28GST recombinante fait l'objet d'essais cliniques pour la vaccination contre la bilharziose (projet Bilhvax). Cette protéine recombinante est donc produite à l'échelle industrielle ; sa production et sa purification, notamment à des fins thérapeutiques, sont donc parfaitement maîtrisées.

En effet, comme décrit plus en détails ultérieurement, le produit selon l'invention permet d'obtenir un effet préventif sur la pathologie précitée. L'administration de la protéine ou du fragment de protéine avant l'épisode inflammatoire de la pathologie permet d'obtenir une diminution de l'inflammation et un basculement de la réponse immunitaire de l'organisme du patient d'une réponse de type Th1, caractéristique de la pathologie, vers une réponse de type Th2, lors d'une poussée de la maladie. C'est le changement de type de réponse immunitaire observé après traitement avec la protéine et/ou le fragment de protéine selon l'invention qui permet de traiter à titre préventif les maladies inflammatoires chroniques de divers organes et même des maladies atteignant un système tout entier, comme par exemple, le système nerveux. On entend par « traitement préventif », tout traitement qui, administré avant la poussée inflammatoire, permet de supprimer une poussée inflammatoire ou de diminuer en intensité et/ou en durée une poussée inflammatoire et/ou qui est capable d'allonger la durée de temps entre deux poussées inflammatoires. L'effet du traitement peut être évalué sur l'état inflammatoire de l'organe ou du système concerné.

On entend par traitement curatif, tout traitement qui administré lors d'une poussée inflammatoire, permet d'atténuer ou de supprimer la poussée, en particulier l'un des symptômes (notamment l'inflammation locale, par exemple de la paroi intestinale) liés à la poussée.

Comme expliqué ultérieurement, le produit selon l'invention agit à travers la diminution de la sécrétion d'interleukines spécifiques d'une réponse inflammatoire de type Th1 et/ou la production accrue d'interleukines correspondant à une réponse immune de type Th2. Ces interleukines circulent dans tout l'organisme et leur diminution ou leur augmentation affecte potentiellement tous les organes.

On verra ultérieurement que le produit selon l'invention permet la production d'interleukines ayant un rôle régulateur (IL-10) dans les maladies chroniques inflammatoires liées à un dérèglement du système immunitaire engendrant une réponse de type Th1 et/ou Th17. Il était connu que l'infestation par des vers parasitaires de type ascaris des patients atteints de sclérose en plaques réduit l'évolution de la maladie (voir « Association between parasite infection and immune responses in multiple sclerosis » Annals of Neurology 2007). En revanche rien n'indiquait qu'une protéine spécifique de *Schistosoma* pouvait avoir un effet curatif et ou préventif sur une pathologie affectant tout le système nerveux d'un organisme. C'est donc le mérite des inventeurs d'avoir testé le produit selon l'invention sur une maladie inflammatoire chronique liée à un dérèglement du système immunitaire appartenant aux maladies neurologiques et affectant tout le système nerveux, comme, notamment, la sclérose en plaques.

Le patient visé par le traitement n'est pas limité selon l'invention. Il peut s'agir, d'un animal, d'un mammifère, plus particulièrement d'un être humain. Il peut également s'agir d'un enfant. Le projet Bilhvax a en effet montré la parfaite innocuité de la protéine recombinante rSh28GST selon l'invention chez les enfants âgés de 5 à 12 ans. Les produits selon l'invention peuvent donc être utilisés pour le traitement des cas pédiatriques des maladies inflammatoires chroniques liées à un dérèglement du système immunitaire, et en particulier des cas pédiatriques de ces maladies. Ainsi, en cas de forte suspicion d'un facteur génétique provoquant ladite maladie ou rendant le patient plus susceptible de développer la maladie (cas d'un enfant dont les deux parents ont développé la maladie de Crohn, par exemple), le produit selon l'invention pourra être administré à titre préventif.

Avantageusement, la composition est administrée à une dose de protéine allant de 50µg/Kg à 1000µg/Kg. La dose peut être égale à 50µg/Kg, 500µg/Kg ou 1000µg/Kg.

La concentration de protéine purifiée administrée par enfant est, par exemple, égale à 253 ug (ce qui correspond à une dose finale de 100 ug du vaccin Bilhvax comprenant la rSh28GST28 plus l'hydroxyde d'aluminium à une concentration de 1 mg/ml): La dose de protéine peut être, par exemple supérieure ou égale à 100 ug et inférieure ou égale à 500 ug de protéine.

Le mode d'administration de la composition de l'invention n'est pas limité selon l'invention. La composition peut être injectée par voie sous-cutanée ou administrée par voie muqueuse, par exemple. La composition selon l'invention peut être administrée, par exemple, sous la forme d'un spray nasal ou buccal, d'un suppositoire, d'un comprimé, d'un lyophilisat, d'une gélule, d'un sirop, d'une solution injectable par voie intraveineuse, sous-cutanée ou intra musculaire, d'une pommade ou gel pour application topique.

La présente invention, ses caractéristiques et les différents avantages qu'elle procure apparaitront mieux à la lecture de la description qui suit et fait référence à des exemples cités à titre non limitatif et aux figures annexées suivantes, sur lesquelles :

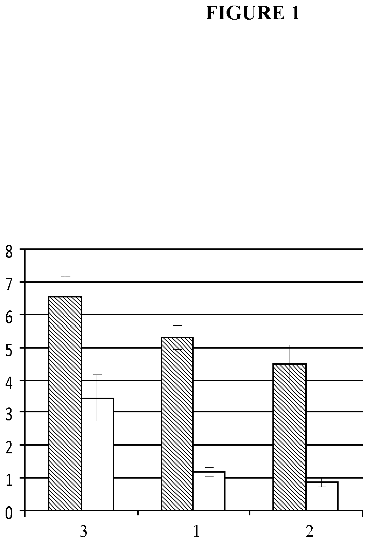

- La figure 1 représente les scores macroscopiques et histologiques obtenus pour différents groupes de rats, le groupe 1 étant le groupe traité avec la protéine de l'invention avant l'induction de la colite, le groupe 2 étant le groupe infecté avec *Schistosoma mansoni* avant l'induction de la colite par le TNBS et le groupe 3 étant le groupe témoin. Le groupe 1 est représenté par le chiffre 1 sur l'axe des abscisses, le groupe 2 est représenté par le chiffre 2 sur l'axe des abscisses et le groupe 3 est représenté par le chiffre 3 sur l'axe des abscisses. Le score macroscopique est représenté par la colonne comportant les rayures diagonales et le score histologique est représenté par la colonne blanche. Les scores macroscopiques et histologiques sont quantifiés par l'axe des ordonnées ;

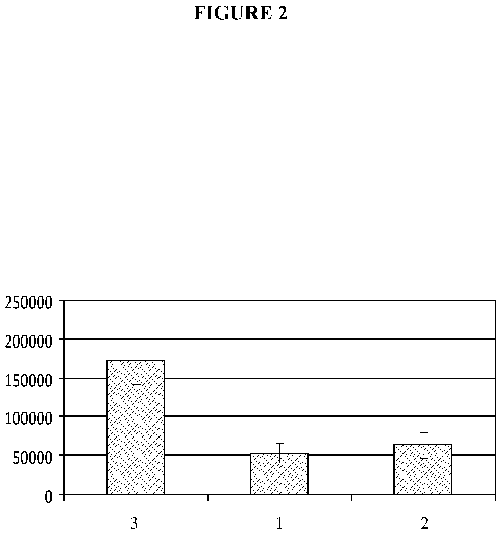

- La figure 2 représente le rapport du taux de myéloperoxidase (MPO) sur le taux de protéines totales dans les groupes de rats cités en référence à la figure 1, le groupe 1 étant représenté par le chiffre 1 sur l'axe des abscisses, le groupe 2 étant représenté par le chiffre 2 sur l'axe des abscisses et le groupe 3 étant représenté par le chiffre 3 sur l'axe des abscisses. L'axe des ordonnées représente le rapport du taux de MPO sur le taux de protéines totales exprimé en ng/mg ;

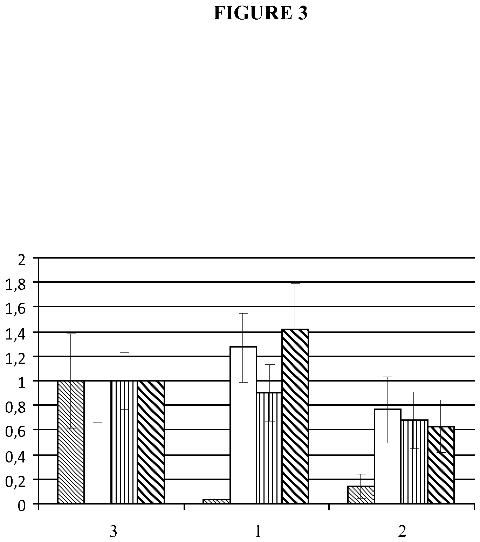

- La figure 3 représente la quantité d'ARN messager codant pour les interleukines IL-1b, IL-13, IL-5 et IL-10 dans les mêmes groupes de rats que ceux utilisés en référence à la figure 1, le groupe 1 étant représenté par le chiffre 1 sur l'axe des abscisses, le groupe 2 étant représenté par le chiffre 2 sur l'axe des abscisses et le groupe 3 étant représenté par le chiffre 3 sur l'axe des abscisses. La quantité d'ARN messager codant pour l'interleukine IL-1b est représentée par les colonnes comportant des fines rayures diagonales, la quantité d'ARN messager codant pour l'interleukine IL-13 est représentée par les colonnes comportant des grosses rayures diagonales, la quantité d'ARN messager codant pour l'interleukine IL-5 est représentée par les colonnes blanches et la quantité d'ARN messager codant pour l'interleukine IL-10 est représentée par les colonnes comportant des rayures verticales. L'axe des ordonnées représente les quantités d'ARN messager en U.A. ;

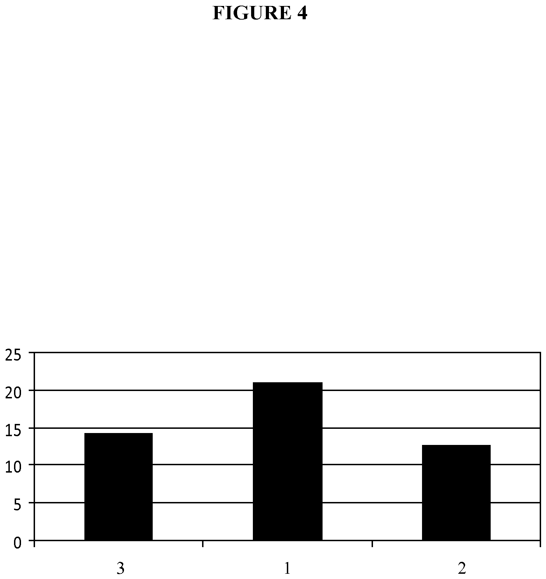

- La figure 4 représente le rapport du taux d'ARN messager codant pour l'arginase de type I (Arg) sur le taux d'ARN messager codant pour l'oxyde nitrique synthétase inductible (iNOS) dans les mêmes groupes de rats que ceux utilisés en référence à la figure 1, le groupe 1 étant représenté par le chiffre 1 sur l'axe des abscisses, le groupe 2 étant représenté par le chiffre 2 sur l'axe des abscisses et le groupe 3 étant représenté par le chiffre 3 sur l'axe des abscisses. L'axe des ordonnées représente le rapport du taux d'ARN messager codant pour l'arginase de type I (Arg) sur le taux d'ARN messager codant pour l'oxyde nitrique synthétase inductible (iNOS) ;

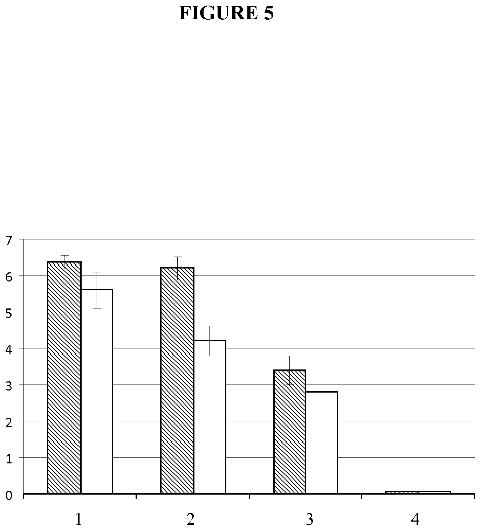

- La figure 5 représente les scores macroscopiques et histologiques obtenus pour différents groupes de souris, le groupe 1 étant le groupe témoin, le groupe 2 étant le groupe traité avec de l'acide 5-aminosalicylique avant l'induction de la colite, le groupe 3 étant le groupe traité avec la protéine selon l'invention avant l'induction de la colite et le groupe 4 étant le groupe qui reçoit une injection d'éthanol avant l'induction de la colite. Le groupe 1 est représenté par le chiffre 1 sur l'axe des abscisses, le groupe 2 est représenté par le chiffre 2 sur l'axe des abscisses, le groupe 3 est représenté par le chiffre 3 sur l'axe des abscisses et le groupe 4 est représenté par le chiffre 4 sur l'axe des abscisses. Le score macroscopique est représenté par les colonnes comportant des rayures diagonales et le score histologique est représenté par les colonnes blanches. Les scores macroscopiques et histologiques sont quantifiés par l'axe des ordonnées ;

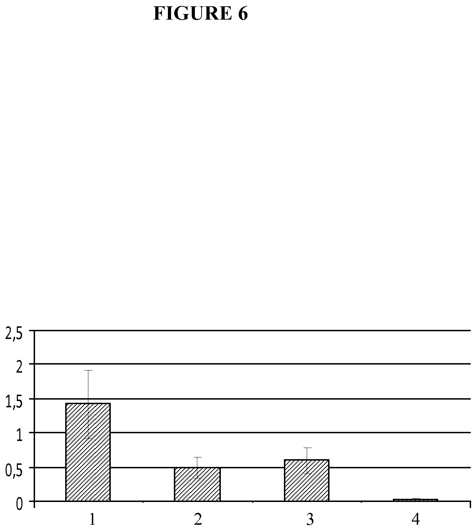

- La figure 6 représente le rapport du taux de myéloperoxidase sur le taux de protéines totales dans les groupes de souris cités en référence à la figure 5, le groupe 1 étant représenté par le chiffre 1 sur l'axe des abscisses, le groupe 2 étant représenté par le chiffre 2 sur l'axe des abscisses, le groupe 3 étant représenté par le chiffre 3 sur l'axe des abscisses et le groupe 4 étant représenté par le chiffre 4 sur l'axe des abscisses. L'axe des ordonnées représente le rapport du taux de MPO sur le taux de protéines totales exprimé en ng/mg ;

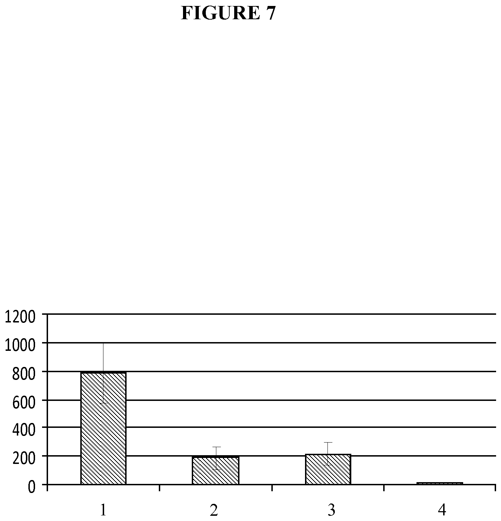

- La figure 7 représente la quantité d'ARN messager codant pour l'interleukine IL-1bdans les mêmes groupes de souris que ceux utilisés en référence à la figure 5, le groupe 1 est représenté par le chiffre 1 sur l'axe des abscisses, le groupe 2 est représenté par le chiffre 2 sur l'axe des abscisses, le groupe 3 est représenté par le chiffre 3 sur l'axe des abscisses et le groupe 4 est représenté par le chiffre 4 sur l'axe des abscisses. L'axe des ordonnées représente la quantité d'ARN messager en U.A. ;

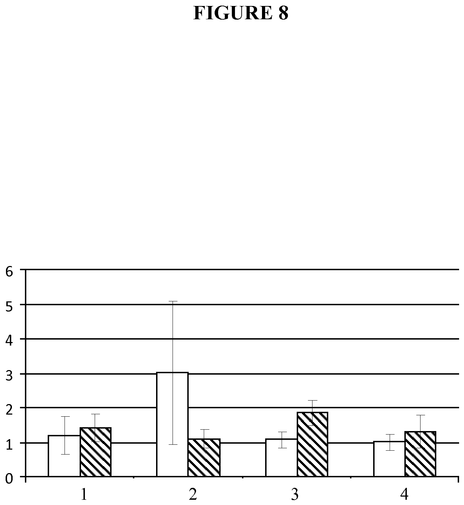

- La figure 8 représente la quantité d'ARN messager codant pour les interleukines IL-5 et IL-13 dans les mêmes groupes de souris que ceux utilisés en référence à la figure 5. Le groupe 1 est représenté par le chiffre 1 sur l'axe des abscisses, le groupe 2 est représenté par le chiffre 2 sur l'axe des abscisses, le groupe 3 est représenté par le chiffre 3 sur l'axe des abscisses et le groupe 4 est représenté par le chiffre 4 sur l'axe des abscisses. La quantité d'ARN messager codant pour l'interleukine IL-5 est représentée par les colonnes blanches et la quantité d'ARN messager codant pour l'interleukine IL-13 est représentée par les colonnes comportant des rayures diagonales. L'axe des ordonnées représente la quantité d'ARN messager en U.A. ;

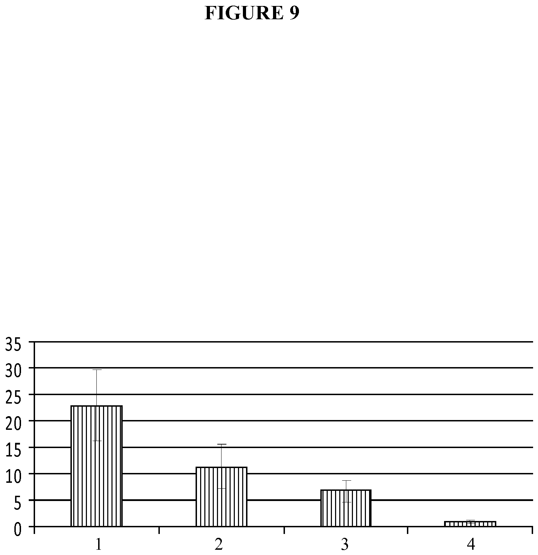

- La figure 9 représente la quantité d'ARN messager codant pour l'interleukine IL-10 dans les mêmes groupes de souris que ceux utilisés en référence à la figure 5. Le groupe 1 est représenté par le chiffre 1 sur l'axe des abscisses, le groupe 2 est représenté par le chiffre 2 sur l'axe des abscisses, le groupe 3 est représenté par le chiffre 3 sur l'axe des abscisses et le groupe 4 est représenté par le chiffre 4 sur l'axe des abscisses. L'axe des ordonnées représente la quantité d'ARN messager en U.A. ;

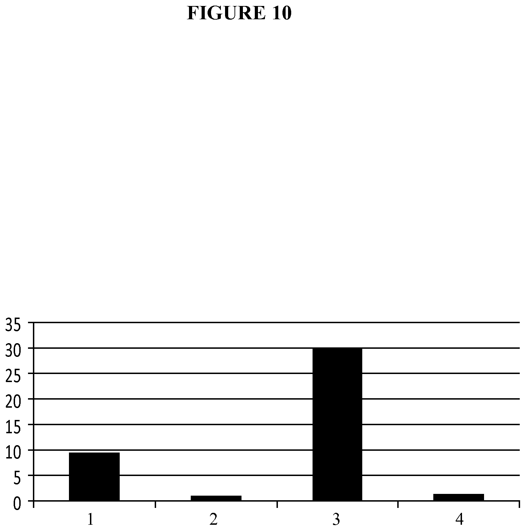

- La figure 10 représente le rapport du taux d'ARN messager codant pour l'arginase de type I sur le taux d'ARN messager codant pour l'oxyde nitrique synthétase inductible dans les mêmes groupes de souris que ceux utilisés en référence à la figure 5. Le groupe 1 est représenté par le chiffre 1 sur l'axe des abscisses, le groupe 2 est représenté par le chiffre 2 sur l'axe des abscisses, le groupe 3 est représenté par le chiffre 3 sur l'axe des abscisses et le groupe 4 est représenté par le chiffre 4 sur l'axe des abscisses. L'axe des ordonnées représente le rapport du taux d'ARN messager codant pour l'arginase de type I sur le taux d'ARN messager codant pour l'oxyde nitrique synthétase inductible ;

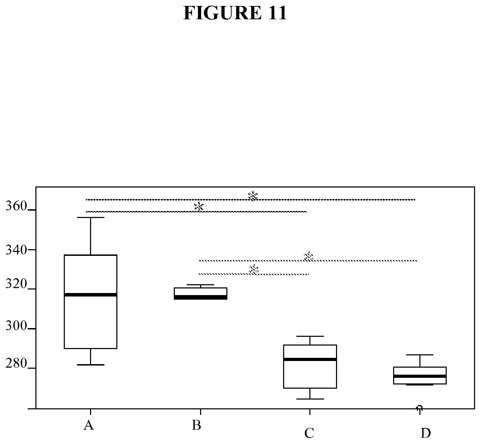

- La figure 11 représente l'analyse des poids des différents groupes de rats à J35 dans l'étude de l'encéphalite allergique expérimentale (modèle de la sclérose en plaques). Le groupe A correspond au groupe de rats ayant reçu deux injections de 5µg/Kg de rSh28GST en combinaison avec de l'hydroxyde d'aluminium (alum) avant l'induction de l'EAE, il est représenté en abscisse par la lettre A, le groupe B correspond au groupe de rats ayant reçu deux injections de 0,5µg/Kg de rSh28GST en combinaison avec l'hydroxyde d'aluminium (alum) avant l'induction de l'EAE, il est représenté en abscisse par la lettre B, le groupe C correspond au groupe de rats ayant reçu deux injections d'hydroxyde d'aluminium seul avant l'induction de l'EAE, il est représenté en abscisse par la lettre C, le groupe D correspond au groupe de rats n'ayant reçu aucune injection avant l'induction de l'EAE, il est représenté sur l'axe des abscisses par la lettre D. L'axe des ordonnées représente le poids des rats des différents groupes.

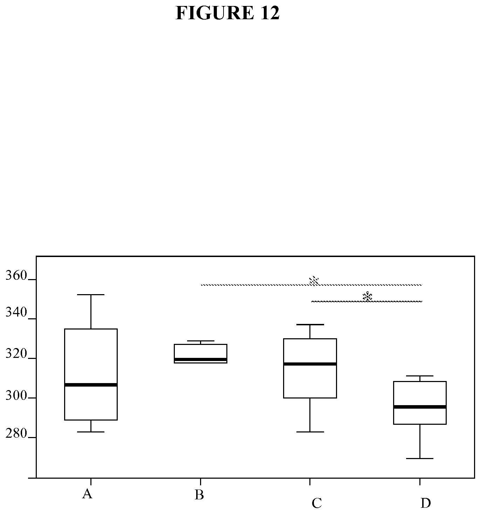

- La figure 12 représente l'analyse des poids (en g) des différents groupes de rats cités en référence à la Figure 11, à J42, les lettres de référence des groupes sont indiquées sur l'axe des abscisses tandis que le poids des rats est indiqué sur l'axe des ordonnées ;

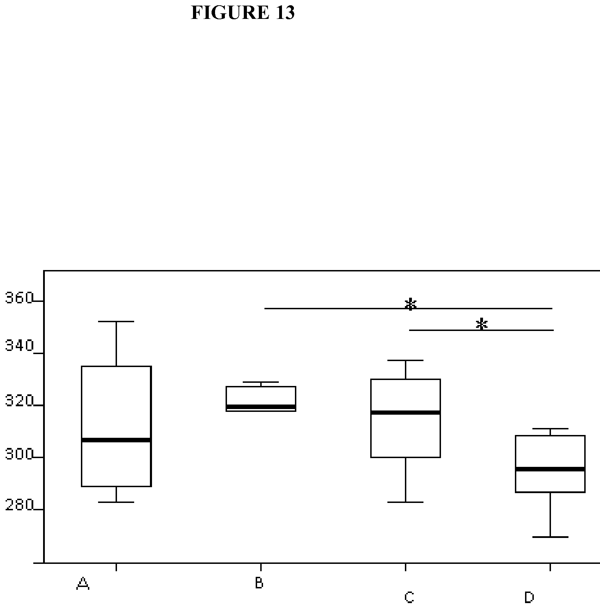

- La figure 13 représente l'analyse des poids (en g) des différents groupes de rats cités en référence à la Figure 11, à J48 les lettres de référence des groupes sont indiquées sur l'axe des abscisses tandis que le poids des rats est indiqué sur l'axe des ordonnées; et

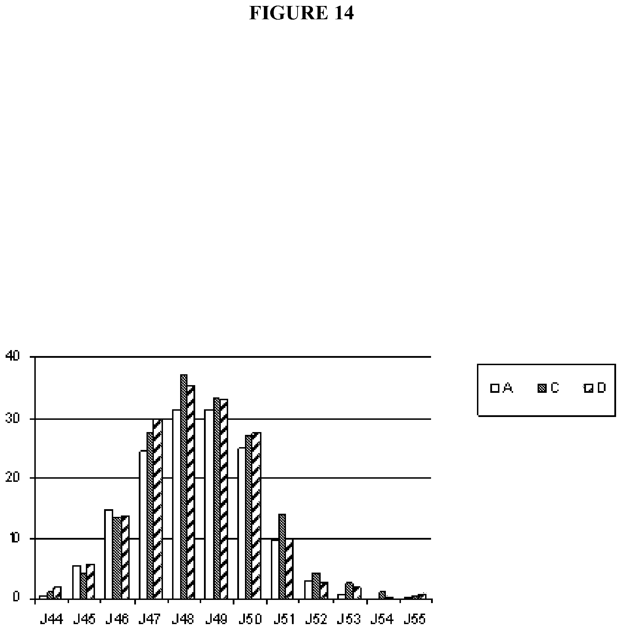

- La figure 14 représente les scores cumulés pour les différents groupes de rats cités en référence à la Fig. 11, les lettres de référence des groupes sont indiquées sur l'axe des abscisses tandis que les scores cumulés sont indiqués sur l'axe des ordonnées ; en référence à la figure 14, les rats des groupes A et C ont reçu trois injections, respectivement à J0, J14 et J28, de 5µg/Kg de rSh28GST en combinaison avec de l'hydroxyde d'aluminium (alum) pour le groupe A et d'alum seul pour le groupe C, avant l'induction de l'EAE à J35 ;

Pour les figures 11 à 13, les résultats sont présentés sous forme de box-and-whisket plots. Les tests statistiques de Kruskal-Wallis et Mann Whitney ont été réalisés afin de déterminer les différences significatives entre les rats des différents groupes. * P < 0.05.

### PARTIE EXPERIMENTALE

### Modèle de la maladie de Crohn

### Protocole expérimental de la colite induite chez le rat par le TNBS (modèle de la maladie de Crohn)

Les animaux utilisés sont des rats Sprague Dawley mâles (180 à 250g). On pèse les animaux puis on supprime la nourriture la veille de manière à ce qu'ils soient à jeun le jour de l'induction de la colite. Les rats sont endormis au pentobarbital (40mg/kg) en injection intra-péritonéale. On injecte intra-rectalement 250µl d'une solution de TNBS (2,4,6 TrinitroBenzene Sulfonic Acid) dilué dans de l'éthanol 100% (volume pour volume) à raison de 80 mg par kg de rat. On évalue la colite 96h après l'injection de TNBS : les animaux sont pesés et sacrifiés avec une injection de 300µl de T61 en intracardiaque. On prélève le colon pour évaluer le score macroscopique. Une section transversale de colon à 4 cm du rectum est également prélevée puis épinglée sur un morceau de liège afin d'y être plongée dans un bain de paraformaldéhyde à 4% pendant 24 heures puis dans un bain d'éthanol à 70% avant déshydratation et inclusion en paraffine pour évaluer le score histologique.

### Protocole expérimental de la colite induite chez la souris par le TNBS (modèle de la maladie de Crohn)

Les animaux sont des souris mâles de fond génétique C57BU6. Elles sont anesthésiées avec un mélange de xylasine-kétamine à la dose de 50mg/kg chacun par voie sous-cutanée. La colite est induite intra-rectalement avec 40µl de solution de TNBS dilué dans de l'éthanol 100% (volume pour volume) à raison de 150 mg par kg de souris. Pour les injections intra-rectales d'éthanol, il s'agit d'éthanol à 50%. (Contrôle solvant).

### Protocole expérimental d'infestation par Schistosoma mansoni

Les rats sont anesthésiés avec un mélange de valium (8mg/kg) et de kétamine (75mg/kg) en injection intra-péritonéale puis rasés au niveau de l'abdomen. Une solution contenant 2000 cercaires (larves infectantes) de *Schistosoma mansoni* est déposée pendant 30 minutes sur l'abdomen des animaux, les cercaires pénètrent par voie transcutanée. Cette infestation représente le « gold standard » de l'expérience.

### Protocole d'étude de la rSh28GST dans la sclérose en plaques (Maladie auto-immune de type Th1)

Ce modèle d'étude de la sclérose en plaques comprend l'injection d'un peptide encéphalitogène de protéines de la myéline (MBP,) pour l'induction d'une maladie auto-immune, appelée EAE (encéphalite allergique expérimentale).

Afin de démontrer le caractère préventif de la rSh28GST, on injecte à rats Lewis de 4 semaines une dose de 5µg/Kg de rSh28GST en mélange avec de l'hydroxyde d'aluminium (voir le paragraphe « préparation de la composition testée »). Quatre semaines plus tard, on induit l'EAE (J=35). On effectue un score clinique à J=44 (première poussée). Le pic de l'EAE se situe à J=48. On observe à J=55, la récupération plus ou moins complète de l'animal. On mesure tout au long du protocole la perte de poids de l'animal, on effectue un score clinique, on quantifie les infiltrats inflammatoires et l'on mesure la démyélinisation à des points critiques (IHC).

### Protocole pour montrer l'effet curatif de la rSh28GST dans le cas de la sclérose en plaques

On utilise un modèle de rat EAE. On immunise les rats Lewis avec une composition de la rSh28GST et de l'hydroxyde d'aluminium en même temps que l'induction de l'EAE, éventuellement à des points critiques de la maladie. La dose injectée est égale à 5µg/Kg, 50µg/Kg, 500µg/Kg et 1000µg/Kg (ces chiffres faisant référence à la dose de rSh28GST, la concentration en hydroxyde d'aluminium étant toujours comme indiqué dans le paragraphe « préparation de la composition testée ») On analyse ensuite les différentes populations cellulaires d'intérêts.

### Protocole d'étude de la polyarthrite rhumatoïde(PR)

On réalise 2 injections de collagène (auto antigène) par voie intradermique à la base de la queue des souris et on mesure l'arthrite de la patte après 21 jours selon un score classique. On effectue ensuite le suivi des paramètres immunologiques (Anticorps, cytokines, cellules régulatrices).

Pour la détermination de l'effet préventif, on injecte avant les deux injections de collagène une dose de 1000µg/Kg de rSh28GST mélangé à de l'hydroxyde d'aluminium (voir le paragraphe « préparation de la composition testée »). Pour démontrer l'effet curatif, on réalise une injection de la composition testée (1000µg/Kg pour la protéine rSh28GST) en même temps que les deux injections de collagène précitées.

### Dosage de la myéloperoxidase (MPO)

Le taux de myéloperoxydase contenue dans les tissus coliques est évalué par la méthode immunologique de type ELISA Sandwich (« Enzyme Linked ImmunoSorbent Assay ») et est rapporté au taux de protéines contenues dans ce même tissu colique.

Le tissu colique (contenant la MPO en cas d'inflammation) est tout d'abord broyé afin d'en obtenir un homogénéisat. Celui-ci est déposé dans un puits de la plaque déjà recouvert avec un anticorps anti-MPO. La réaction antigène (ici MPO)-anticorps anti-MPO se réalise alors. Un second anticorps dirigé contre la molécule de MPO et marqué par la biotine est ensuite ajouté et est révélé grâce à l'addition de streptavidine (très affine pour la biotine) couplée avec un chromogène. En présence de son substrat, ce dernier libère une substance colorée dont la densité optique est évaluée par un spectrophotomètre. La densité optique mesurée est proportionnelle à la quantité de protéine MPO contenue dans l'homogénéisat du tissu colique.

### Préparation de la composition testée (protéine rSh28GST+ hydroxyde d'aluminium)

La protéine rSh28GST utilisée est la protéine recombinante produite dans *Saccharomyces cerevisiae* et fabriquée par la société Eurogentec. La séquence totale du gène représentée par la séquence SEQ ID NO : 4 est utilisée. La protéine se présente sous forme lyophilisée, une fiole contient 165µg (+ ou - 5%) de protéines. La composition pharmaceutique est reconstituée avec 660µl de gel d'hydroxyde d'aluminium (2%) dilué au 10^{ème} dans du sérum physiologique dans une fiole de protéine d'où une concentration de 250µg/ml. La composition ainsi obtenue est injectée en sous-cutanée aux animaux à la même concentration. C'est ce mélange rSh28GST + hydroxyde d'aluminium qui est injecté aux animaux dans tous les protocoles expérimentaux. Les doses indiquées correspondent à la dose en protéine rSh28GST.

### Rapport « Arginase I/ Nitric Oxyde Synthétase 2 »

Le rapport représente le taux d'ARN messager codant pour l'arginase I (Arg) sur le taux d'ARN messager codant pour la nitric oxyde synthétase inductible (iNOS) de type 2 pour un échantillon colique donné. Le taux d'ARN messager est obtenu par une méthode de réaction en chaine par polymérase en temps réel (RT-PCR). Il s'agit d'une technique de biologie moléculaire d'amplification in vitro d'ADN. A chaque cycle d'amplification, la quantité d'ADN total est mesurée grâce à une sonde fluorescente ; la cinétique de l'ensemble des cycles d'amplification permet d'obtenir une quantification de l'ARN messager initial.

### Expression de l'ARN messager des interleukines IL-1b, IL-13, IL-5 et IL-10

La présence et la quantification de l'ARN messager de différentes interleukines dans les tissus coliques sont obtenues en utilisant la méthode de PCR en temps réel expliquée au point précédent.

### Résultats expérimentaux chez le rat

Trois groupes de rats Sprague Dawley sont constitués. Le groupe 1 reçoit 2 injections par voie sous cutanée d'une composition telle que précitée (rSh28GST+hydroxyde d'aluminium) à une dose de 50µg/kg (pour la protéine rSh28GST) la première à J=0 et la seconde à J=28. Le groupe 2 (« gold standard ») est infesté par 2000 cercaires de *Schistosoma mansoni* à J=0. Le groupe 3 est le groupe témoin. A J=35, la colite est induite chez les rats des trois groupes par une injection intra-rectale de TNBS, puis le sacrifice a lieu à J=39.

### Scores macroscopique et histologique

Comme représenté sur la figure 1, on constate que l'inflammation de la paroi intestinale des rats des groupes 1 et 2 est diminuée par rapport à celle des rats du groupe 3. De plus, le score histologique confirme le score macroscopique.

### Dosage de la myéloperoxidase (MPO)

La myéloperoxydase est une enzyme présente dans les granulocytes neutrophiles et également un marqueur d'inflammation. Comme représenté sur la figure 2, on remarque que la quantité de MPO dans les tissus coliques des rats du groupe 1 et du groupe 2 est réduite par rapport à la quantité présente dans les tissus coliques des rats du groupe 3. Ces résultats montrent que l'injection de la protéine rSh28GST permet de réduire de manière significative la sécrétion de MPO lors d'une colite inflammatoire.

### Interleukines IL-1b, IL-13, IL-5 et IL-10

L'IL-1b est une interleukine secrétée lors d'une réponse immunitaire inflammatoire de type Th1. Comme représenté sur la figure 3, on constate que la quantité d'ARN messager codant pour cette interleukine est réduite dans les tissus coliques des rats des groupes 1 et 2 (plus encore dans le groupe 1 que dans le groupe 2). Ces résultats montrent que l'injection de la protéine rSh28GST réduit la réponse immunitaire de type Th1 de manière plus prononcée que l'infection par *Schistosoma mansoni à* J0.

Les interleukines IL-5 et IL-13 sont sécrétées lors d'une réponse immunitaire de type Th2. Comme représenté sur la figure 3, la quantité d'ARN messager codant pour ces deux interleukines est augmentée dans les tissus coliques des rats du groupe 1 par rapport aux tissus des rats du groupe 3. La quantité d'ARN messager codant l'IL-5 et l'IL-13 est plus importante dans le groupe 1 que dans le groupe 2. Ces résultats montrent que la protéine rSh28GST induit une forte expression de l'ARN messager de l'IL-5 et de l'IL-13. La protéine rSh28GST permet donc d'induire une réponse immunitaire de type Th2 qui contrebalance la réponse immunitaire de type Th1 retrouvée dans le cas de la colite induite au TNBS.

Il a été démontré que des patients atteints de maladie de Crohn réagissaient favorablement à un traitement à base d'interleukine 10 (voir Braat H, Rottiers P, Hommes DW, Huyghebaert N, Remaut E, Remon JP, van Deventer SJ, Neiryncjk S, Peppenlenbosch MP, Steidler L (June 2006) « A phase I trial with transgenic bacteria expressing Interleukin-10 in Crohn's disease » Clin. Gastroenterol. Hepatol.4 (6) : 754-9. doi :10.1016/j.cgh.2006.03.028. PMID 16716759). Comme représenté sur la figure 3, la présence d'ARN messager codant pour l'IL-10 dans les tissus coliques des rats du groupe 1 est plus importante que celle des rats du groupe 2. Ces résultats montrent que la protéine rSh28GST induit la production d'ARN messager codant pour l'IL-10, ce qui est un facteur favorable dans le cas du traitement curatif de la maladie de Crohn. Par ailleurs, l'IL-10 est sécrétée lors d'une réponse immunitaire de type Th2 ce qui concorde avec les résultats illustrés dans la Figure 3.

### Rapport « Arginase I/ Nitric Oxyde Synthétase 2 »

L'arginase de type I est une enzyme, utilisée ici comme marqueur, qui caractérise les macrophages M2 dits alternatifs, présents dans des réactions immunitaires anti-inflammatoires Th1 ; la nitric oxyde synthétase de type 2 est elle aussi une enzyme, mais utilisée comme marqueur caractérisant les macrophages dits classiques présents dans des réactions immunitaires pro-inflammatoires Th2. Le rapport entre ces deux enzymes reflète la présence au sein des tissus coliques de tel ou tel type de macrophages, et permet d'apprécier la balance entre les deux réponses immunitaires Th1 et Th2.

Comme représenté sur la figure 4, la protéine rSh28GST permet de favoriser la réponse de type Th2 qui vient ainsi contrebalancer la réponse de type Th1 induite par la colite.

### Résultats expérimentaux chez la souris.

Les 2 modèles de Rongeurs utilisés (rats et souris) sont des modèles expérimentaux classiques et largement décrits dans la littérature comme modèles de la maladie de Crohn. La souche de souris utilisée ici (C57 BL6) est plutôt pro-Th1 et donc l'obtention de résultats diminuant une réponse de type Th1 dans ce modèle animal est encore plus probante que chez le rat.

Quatre groupes de souris sont constitués. Le groupe 1 ne reçoit aucun traitement, seulement une colite au TNBS à J=35 : c'est le groupe de contrôle. Les souris du groupe 2 sont nourries à partir de J=30 avec des croquettes contenant du 5-ASA : acide 5-aminosalicylique à raison de 1 g de 5-ASA pour 600 g de croquettes, puis une colite au TNBS est induite à J=35. Les souris du groupe 3 reçoivent deux injections de composition telle que précitée (rSh28GST+ hydroxyde d'aluminium) à la dose de 1000µg/kg (pour la rSh28GST) à J0 et J28, puis une injection intra-rectale de TNBS. Les souris du groupe 4 ne reçoivent qu'une injection intra-rectale d'éthanol à 50% à J35.(Contrôle Solvant du TNBS). Les souris des quatre groupes sont sacrifiées à J=37.

### Score macroscopique et histologique

Comme représenté sur la figure 5, le score macroscopique qui reflète l'inflammation est fortement diminué chez les souris du groupe 3. Les résultats obtenus sont meilleurs que ceux obtenus avec le 5-ASA. Le score histologique confirme le score macroscopique.

### Dosage de la myéloperoxidase (MPO)

La figure 6 montre que la rSh28GST diminue, dans le cas d'une colite, le taux de MPO au niveau des tissus coliques des souris du groupe 3. Dans le cas où les souris n'ont pas reçu de colite (groupe 4), l'activité de la MPO est inchangée.

### Interleukines IL-1b, IL-13, IL-5 et IL-10

Les figures 7 à 9 confirment, chez la souris, les résultats obtenus chez le rat. La protéine rSh28GST réduit le taux d'ARN messager codant pour l'IL-1b et augmente celui de l'IL-13 et de l'IL-5 en cas de colite. La protéine précitée semble donc avoir un effet préventif du fait de l'augmentation de la production d'IL-10.

### Rapport « Arginase de type I/Nitric Oxyde Synthétase 2 »

La figure 10 montre, comme dans le cas du rat, une augmentation du rapport du taux d'ARN messager codant pour l'arginase de type I sur celui codant pour la nitric oxyde synthétase 2 dans le cas du groupe 3 par rapport aux groupes 1, 2 et 4. Cette augmentation du rapport « Arginase de type I/Nitric Oxyde Synthétase 2 » est significative d'une augmentation de la présence de macrophages associés à une réponse Th2. La composition de l'invention permet donc de favoriser une réponse de type Th2 dans le cas de la colite induite, ce qui n'est pas le cas de l'acide 5-aminosalicylique (voir groupe 2).

Par ailleurs, un autre groupe de souris a subi les deux injections de rSh28GST tel que précité et une simple injection intra-rectale d'éthanol à la place du TNBS. On teste ainsi l'influence de la protéine sans inflammation. Dans ce groupe, dont les résultats ne sont pas illustrés sur les figures, l'activité de la MPO est inchangée. La protéine de l'invention réduit donc l'inflammation mais ne modifie pas les taux de MPO lorsqu'il n'y a pas d'inflammation induite. Les résultats de ce groupe montrent aussi que, sans inflammation induite, la protéine seule permet d'augmenter l'ARN messager codant pour l'IL-13, l'IL-5 et l'IL-10 et que sans inflammation induite, la protéine est sans activité sur les macrophages.

### Modèle de la sclérose en plaques

Le modèle utilisé est le modèle de l'encéphalite allergique expérimentale (EAE). Dans le cas présent, l'EAE est induite par une injection de protéine basique de la myéline (MBP).

L'effet de l'immunisation par la rSh28GST sur le développement de l'EAE induite par la MBP dans un modèle de rats Lewis a été étudié.

### Matériels et méthodes :

### 1- Réactifs :

### a) Préparation de rSh28GST(alum): (Lot : GLP. Désigné par : L-Bix-P08/165a). Le terme « alum » désigne une suspension aqueuse d'hydroxyde d'aluminium comme décrit plus en détails ci-dessous.

### Dose de 5µg/kg (+ alum) :

On dissout 1 fiole contenant 165µg de rSh28GST dans 660µl d'eau physiologique pour obtenir une solution de 250 µg/ml. On prépare ensuite une solution à 10 µg/ml : dilution au 1:25ème en eau physiologique. On ajoute l'alum : 10 µl (« alum » désigne alhydrogel 2% VacciGrade_50 ml cat : vac-alu-50 lot : AHG-33-427)

### Dose de 0,5 µg/kg (+ alum) :

A partir de la solution de rSh28GST à 250 µg/ml, on prépare une solution à 1 µg/ml : dilution au 1:250eme en eau physiologique. On ajoute l'alum : 10 µl (alum désigne alhydrogel 2% VacciGrade_50 ml cat : vac-alu-50 lot : AHG-33-427)

### b) Dose alum :

La dose d'alum est obtenue en diluant 10 µl d'alum à 2,5 ml d'eau physiologique (l'alum désigne alhydrogel 2% VacciGrade_50 ml cat : vac-alu-50 lot : AHG-33-427)

### c) Préparation de l'émulsion destinée à induire l'EAE

On prépare une solution à 100 µg/ml, soit 3 ml de CFA à 2 mg/ml + 1 ml de IFA + 4 ml d'une solution MBP à 200 µg/ml dans du PBS. Deux doses de 8 ml équivalent à 50 µl/5 µg de MBP sont injectées au niveau des pattes du rat. Au total, on injecte 100 µl/10µg MBP par rat.

La solution de MBP dans du PBS précitée est obtenue par dilution de MBP (lot 2011) : 1064 µl à 940 µg/ml dans 3,94 ml de perborate de sodium (PBS).

CFA désigne l'adjuvant complet de Freund c'est-à-dire comprenant des mycobactéries (*M. tuberculosis*). IFA désigne la forme incomplète de l'adjuvant de Freund. 1 mL d'adjuvant de Freund dans sa forme incomplète est constitué de 0.85 mL d'huile de paraffine et 0.15 mL monooleate de mannide. Dans sa forme complète, il comprend en outre une quantité donnée de *Mycobacterium tuberculosis* (H37Ra, ATCC 25177) thermo-inactivé (tué thermiquement) et séché.

CFA à 2 mg/ml de M. *tuberculosis* : IFA Sigma (réf. F5506 lot : 070M8706) et *Mycobacterium tuberculosis* H37 RA Difco (réf. 3114-25 lot. 131582 L-00535-02) de manière à obtenir la concentration de 2mg/ml précitée.
Anesthésiques fournis par la zootechnie : kétamine et xylazine

### d. Animaux :

Rats mâles Lewis âgés de 6 semaines (CERJ élevage Janvier) au début des expériences et immunisés par la MBP à 12 semaines (n=10 rats/groupe). Les injections sont effectuées en sous-cutanée dans le cou du rat. 40 rats ont été utilisés.

### Protocole expérimental

L'expérimentation débute à la septième semaine de vie des rats.

A J=0 les 10 rats du groupe A reçoivent une injection de la solution précitée contenant 5 µg/kg de rSh28GST et de l'alum. A J=0 les 10 rats du groupe B reçoivent une injection de la solution précitée contenant 0,5 µg/kg de rSh28GST et de l'alum. A J=0 les 10 rats du groupe C reçoivent une injection de la solution alum précitée seule ; ils reçoivent une seconde injection d'alum seul à J=28. A J=0 les 10 rats du groupe D qui est le groupe de contrôle ne reçoivent aucune injection. A J=28, les rats des groupes A et B reçoivent une 2ème injection de rSh28GST(alum) au même dosage que la première injection

A J=35, on induit l'EAE par une injection contenant de la protéine de base de la myéline (voir paragraphe c)). L'étude clinique débute à J44 et se termine à J56 par le sacrifice des animaux.

### Réponse humorale des rats immunisés par la P28 GST

Le dosage des anticorps IgG anti-rSh28GST a été réalisé sur les prélèvements sanguins effectués à J35 (jour de l'induction de l'EAE) et J56 (jour du sacrifice) par technique Elisa. Les résultats révèlent que seuls 6 rats (sur 10) du groupe A et 4 rats (sur 10) du groupe B ont présenté une réponse immunitaire détectable tout au long du protocole clinique. Seuls ces rats présentant une réponse immune sous forme de production détectable d'anticorps IgG anti-rSh28GST à la rSh28GST à J35 et à J56 ont été inclus dans l'étude qui suit concernant les poids des rats..

### Etude des variations du poids des rats dans les quatre groupes

Le poids des animaux a été évalué le jour de l'induction de l'EAE (soit 35 jours après la première injection de rSh28GST et tous les jours à partir des premiers symptômes (J42). Les résultats obtenus indiquent qu'une perte poids d'environ 10% pour tous les groupes est observée au moment de l'acmé de la maladie. Néanmoins, des tests statistiques non paramétriques ont été effectués sur ces données et ont mis en évidence des différences significatives entre les groupes.

Ainsi, à J35, les rats des groupes A et B (quelle que soit la dose de P28GST) présentent un poids significativement plus important par rapport aux rats des groupes C et D.

Ces résultats sont visibles sur la fig. 11 qui représente l'analyse des poids des différents groupes de rats à J35. Les résultats sont présentés sous forme de box-and-whisket plots. Les tests statistiques de Kruskal-Wallis et Mann Whitney ont été réalisés afin de déterminer les différences significatives entre les rats des différents groupes. * P < 0.05.

De plus, il apparait qu'à J42 et à J48, les rats malades du groupe D présentent une masse corporelle inférieure à ceux des rats des groupes A et B.

La fig. 12 représente l'analyse des poids des différents groupes de rats à J42. La fig. 13 représente l'analyse des poids des différents groupes de rats à J48. Pour les deux figures, les résultats sont présentés sous forme de box-and-whisket plots. Les tests statistiques de Kruskal-Wallis et Mann Whitney ont été utilisés pour déterminer les différences significatives entre les rats ayant reçu 2 doses de rSh28GST(Alum) à 5µg/kg, 0.5 µg/kg, alum seul et témoin. * P < 0.05.

### Scores Cliniques

Pour l'évaluation du score clinique, d'autres groupes de rats ont été utilisés (n=22 pour le groupe A, n=24 pour le groupe C et n=8 pour le groupe D). Le protocole est le même que précité à la différence que les rats du groupe A ont reçu trois injections (respectivement à J0, J14 et J28) de 5µg/Kg de rSh28GST en combinaison avec de l'hydroxyde d'aluminium (alum) (de manière à obtenir une réponse immune) et que les rats du groupe C ont reçu trois injections (à J0, J14 et J28) d'alum seul. L'EAE a été induite à J35. Les rats ont été scorés quotidiennement à partir des premiers symptômes (J44) jusqu'en fin de phase de rémission (J55). Des scores cliniques ont été attribués selon l'échelle de référence suivante : score de 0 = aucun symptôme ; score de 0.5 = faiblesse musculaire dans la queue ; score de 1 = queue hypotonique ; score de 2 = queue hypotonique + faiblesses musculaires dans les membres postérieurs ; score de 3 = 1 membre postérieur paralysé ; score de 4 = 2 membres postérieurs paralysés, score 5 = les 2 membres postérieurs paralysés + une incapacité à se nettoyer et score de 6 = mort de l'animal. Ainsi, la Fig. 14 représente les scores cumulés observés pour les différents groupes de rats précités en fonction de la durée post immunisation par la rSh28GST. Les groupes sont représentés en abscisse tandis que le score cumulé est indiqué en ordonnée. Les rats du groupe A débutent la poussée à J44 avec une acmé atteinte à J46 suivant la même cinétique que les groupes C et D mais les scores cumulés à partir de J45 sont moins importants que ceux des groupes C et D, notamment entre J45 et J55. Les diminutions du score cumulé sont significatives à J45 et J48. Les rats ayant développé une réponse anticorps à la dose de 5 µg/ kg (groupe A) manifestent donc une diminution des scores cliniques cumulés. Par ailleurs, la mortalité dans le groupe A est nulle, tandis qu'un rat du groupe contrôle D est mort ainsi que trois rats du groupe C (alum seul).

## Revendications

1. Produit choisi parmi une protéine, un fragment de ladite protéine, une séquence dérivée et une séquence homologue de ladite protéine, **caractérisé en ce que** :
- ladite protéine comprend ou est constituée par la protéine 28KDa Glutathion S-transférase d'un schistosome choisi parmi *Schistosoma haematobium, Schistosoma mansoni, Schistosoma bovis* représentée respectivement par les séquences SEQ ID NO : 1, SEQ ID NO : 2, et SEQ ID NO : 3 ;
- ledit fragment est un fragment d'une desdites protéines comportant, au moins une région comprise parmi les régions suivantes : la région comprise de l'acide aminé en position 15 à l'acide aminé en position 60, la région comprise de l'acide aminé en position 20 à l'acide aminé en position 50, la région comprise de l'acide aminé en position 24 à l'acide aminé en position 43, la région comprise de l'acide aminé en position 170 à l'acide aminé en position 220, la région comprise de l'acide aminé en position 180 à l'acide aminé en position 215, la région comprise de l'acide aminé en position 190 à l'acide aminé en position 211 de la séquence SEQ ID NO : 1, SEQ ID NO : 2 ou SEQ ID NO : 3 ; ceci induisant une réponse immunitaire de type Th2, **caractérisée par** la production d'IL-4, d'IL-5 ou d'IL-13 par des lymphocytes ne produisant pas d'IFN gamma ;
et ceci diminuant la réponse immunitaire de type Th1, la réponse immunitaire de type Th1 étant **caractérisée par** la production d'IFN gamma et de TNF par des lymphocytes ne produisant pas d'IL-4 ou d'IL-5, et la production d'IL-1b par les tissus coliques de rat ;
- ladite séquence dérivée est dérivée d'une desdites séquences SEQ ID NO : 1, SEQ ID NO : 2 et SEQ ID NO : 3 ou d'un desdits fragments précités et obtenue notamment par suppression, substitution ou ajout d'un ou plusieurs acides aminés, dans la séquence SEQ ID NO : 1, SEQ ID NO : 2 ou SEQ ID NO : 3, ladite séquence dérivée comprenant au moins une région comprise parmi les régions suivantes : la région comprise de l'acide aminé en position 15 à l'acide aminé en position 60, la région comprise de l'acide aminé en position 20 à l'acide aminé en position 50, la région comprise de l'acide aminé en position 24 à l'acide aminé en position 43, la région comprise de l'acide aminé en position 170 à l'acide aminé en position 220, la région comprise de l'acide aminé en position 180 à l'acide aminé en position 215 et la région comprise de l'acide aminé en position 190 à l'acide aminé en position 211 de la séquence SEQ ID NO : 1, SEQ ID NO : 2 ou SEQ ID NO : 3 ; ceci entraînant l'induction d'une réponse Th2 et/ou la diminution de la réponse Th1, caractérisées respectivement par la production d'IL-4, d'IL-5 ou d'IL-13, et la production d'IFN gamma, de TNF ou d'IL-1b ; et
- ladite séquence homologue est une séquence homologue d'une desdites séquence SEQ ID NO: 1, SEQ ID NO: 2 et SEQ ID NO: 3 ou d'un desdits fragments, et présente une identité d'au moins 80% ou d'au moins 85% avec au moins l'une des régions suivantes desdites séquences : la région comprise de l'acide aminé en position 15 à l'acide aminé en position 60, la région comprise de l'acide aminé en position 20 à l'acide aminé en position 50, la région comprise de l'acide aminé en position 24 à l'acide aminé en position 43, la région comprise de l'acide aminé en position 170 à l'acide aminé en position 220, la région comprise de l'acide aminé en position 180 à l'acide aminé en position 215, et la région comprise de l'acide aminé en position 190 à l'acide aminé en position 211 de la séquence SEQ ID NO : 1, SEQ ID NO : 2 ou SEQ ID NO : 3 ; ou avec la totalité de l'une des séquences SEQ ID NO : 1, SEQ ID NO : 2 ou SEQ ID NO : 3 ; ceci entraînant l'induction d'une réponse Th2 et/ou la diminution de la réponse Th1, caractérisées respectivement par la production d'IL-4, d'IL-5 ou d'IL-13, et la production d'IFN gamma, de TNF ou d'IL-1b;
pour son utilisation dans le traitement préventif et/ou curatif d'une maladie inflammatoire chronique liée à un dérèglement du système immunitaire générant une réponse immunitaire de type Th1 et /ou Th17 choisie parmi les maladies inflammatoires auto-immunes comprenant la maladie de Berger, la maladie de Basedow, la thyroïdite d'Hashimoto, le myxoedème primaire, la maladie coeliaque, la rectocolite hémorragique, la maladie de Crohn, l'arthrite rhumatoïde, la cirrhose biliaire primitive, la cholangite sclérosante primitive, les anémies hémolytiques auto-immunes, l'anémie de Biermer (Anémie pernicieuse), le lupus érythémateux, le syndrome de CREST, le diabète de type 1, la sclérodermie, le pemphigus vulgaire, la pemphigoïde bulleuse, l'épidermolyse Bulleuse Acquise, la dermatite herpétiforme, la myasthénie, le syndrome myasthénique de Lambert-Eaton, la polymyosite, le syndrome de Goujerot-Sjögren, la sclérose en plaques, la polyarthrite rhumatoïde, la maladie de Graves et le psoriasis.

2. Produit pour son utilisation selon la revendication 1 **caractérisé en ce que** ladite protéine est représentée par la séquence SEQ ID NO : 1 (28 GST glutathion transférase de *Schistosoma haematobium),* ledit fragment est un fragment de ladite protéine comprenant la région comprise de l'acide aminé en position 15 à l'acide aminé en position 60, ou la région comprise de l'acide aminé 20 à l'acide aminé 50, ou la région comprise de l'acide aminé en position 24 à l'acide aminé en position 43 et la région comprise de l'acide aminé en position 170 à l'acide aminé en position 220, ou la région comprise de l'acide aminé en position 180 à l'acide aminé en position 215, ou la région comprise de l'acide aminé en position 190 à l'acide aminé en position 211, ladite séquence dérivée est obtenue à partir de SEQ ID NO : 1 et ladite séquence homologue présente une identité d'au moins 80% ou d'au moins 85% avec la séquence SEQ ID NO : 1.

3. Produit pour son utilisation selon la revendication 2, **caractérisé en ce qu'**il est le produit d'expression d'une séquence nucléotidique codant :
- pour la protéine 28KDa Glutathion-S-transférase représentée par SEQ ID NO : 1 ; ou
- pour un fragment de ladite protéine représentée par SEQ ID NO : 1, ledit fragment comportant ou étant constitué d'au moins la région comprise de l'acide aminé en position 15 à l'acide aminé en position 60, ou la région comprise de l'acide aminé en position 20 à l'acide aminé en position 50, ou la région comprise de l'acide aminé en position 24 à l'acide aminé en position 43 et la région comprise de l'acide aminé en position 170 à l'acide aminé en position 220, ou la région comprise de l'acide aminé en position 180 à l'acide aminé en position 215, ou la région comprise de l'acide aminé en position 190 à l'acide aminé en position 211 de la séquence SEQ ID NO : 1 ; ou
- pour une desdites séquences dérivées ; ou
- pour une desdites séquences homologues ;
ladite séquence nucléotidique correspondant à la séquence SEQ ID NO : 4 codant pour SEQ ID NO : 1.

4. Produit pour son utilisation selon la revendication 3, **caractérisé en ce qu'**il est le produit d'expression de ladite séquence codante SEQ ID NO : 4 dans *Saccharomyces cerevisiae* ou dans *Escherichia coli.*

5. Produit pour son utilisation selon l'une quelconque des revendications 1 à 4, pour son utilisation chez un enfant.

6. Composition pharmaceutique pour son utilisation selon l'une quelconque des revendications 1 et 5, ladite composition comprenant, en tant qu'agent actif, un produit tel que défini dans l'une quelconque des revendications 1 à 4 et un excipient pharmacologiquement acceptable.

7. Composition pharmaceutique pour son utilisation selon la revendication 6, pour le traitement préventif et/ou curatif d'une maladie choisie parmi les maladies inflammatoires auto-immunes comprenant la maladie de Berger, la maladie de Basedow, la thyroïdite d'Hashimoto, le myxoedème primaire, la maladie coeliaque, la rectocolite hémorragique, la maladie de Crohn, l'arthrite rhumatoïde, la cirrhose biliaire primitive, la cholangite sclérosante primitive, les anémies hémolytiques auto-immunes, l'anémie de Biermer (Anémie pernicieuse), le lupus érythémateux, le syndrome de CREST, le diabète de type 1, la sclérodermie, le pemphigus vulgaire, la pemphigoïde bulleuse, l'épidermolyse Bulleuse Acquise, la dermatite herpétiforme, la myasthénie, le syndrome myasthénique de Lambert-Eaton, la polymyosite, le syndrome de Goujerot-Sjögren, la sclérose en plaques, la polyarthrite rhumatoïde, la maladie de Graves et le psoriasis et les éventuelles formes pédiatriques de ces maladies.

8. Composition pour son utilisation selon l'une quelconque des revendications 1 ou 6, **caractérisée en ce qu'**elle comprend un adjuvant choisi parmi les sels d'aluminium.

9. Composition pharmaceutique pour son utilisation selon l'une quelconque des revendications 1, 6 et 7, **caractérisée en ce qu'**elle comprend comme adjuvant l'hydroxyde d'aluminium.

10. Composition pour son utilisation selon la revendication 9, **caractérisée en ce que** la concentration en adjuvant est comprise de 0,5 mg/ml à 2 mg/ml.

11. Composition pour son utilisation selon la revendication 10, **caractérisée en ce que** la concentration en adjuvant est comprise de 0,3 mg/ml à 1 mg/ml.

12. Composition pour son utilisation selon la revendication 10, **caractérisée en ce que** la concentration en adjuvant est comprise de 220 µg/ml à 280 µg/ml.

13. Composition pour son utilisation selon la revendication 10, **caractérisée en ce que** la concentration en adjuvant est sensiblement égale à 250 µg/ml.

## Patentansprüche

1. Produkt ausgewählt aus einem Protein, einem Proteinfragment, einer abgeleiteten Proteinsequenz und einer homologen Proteinsequenz, **dadurch gekennzeichnet, dass**:
- das Protein das 28-Kda-Glutathion-S-Transferaseprotein eines Schistosomen, ausgewählt aus *Schistosoma haematobium, Schistosoma mansoni, Schistosoma bovis,* dargestellt durch die Sequenzen SEQ ID NO: 1, SEQ ID NO: 2 bzw. SEQ ID NO: 3, umfasst oder daraus besteht;
- wobei das Fragment ein Fragment eines der Proteine ist, die mindestens eine Region aus den folgenden Regionen umfassen: der Region im Bereich von der Aminosäure an Position 15 bis zur Aminosäure an Position 60, der Region im Bereich von der Aminosäure an Position 20 bis zur Aminosäure an Position 50, der Region im Bereich von der Aminosäure an Position 24 bis zur Aminosäure an Position 43, der Region im Bereich von der Aminosäure an Position 170 bis zur Aminosäure an Position 220, der Region im Bereich von der Aminosäure an Position 180 bis zur Aminosäure an Position 215, der Region im Bereich von der Aminosäure an Position 190 bis zur Aminosäure an Position 211 der Sequenz SEQ ID NO:1, SEQ ID NO:2 oder SEQ ID NO:3; wodurch eine Th2-Immunreaktion induziert wird, die durch die Produktion von IL-4, IL-5 oder IL-13 durch Lymphozyten, die kein IFN-gamma produzieren, gekennzeichnet ist;
und wodurch die Th1-Immunreaktion verringert wird, wobei die Th1-Immunreaktion durch die Produktion von IFN-gamma und von TNF durch Lymphozyten, die kein IL-4 oder IL-5 produzieren, und die Produktion von IL-1b durch Rattenkolitisgewebe gekennzeichnet ist;
- wobei die abgeleitete Sequenz von einer der Sequenzen SEQ ID NO:1, SEQ ID NO:2 und SEQ ID NO:3 oder einer der vorgenannten Fragmente abgeleitet ist und insbesondere durch Suppression, Substitution oder Addition einer oder mehrerer Aminosäuren in der Sequenz SEQ ID NO:1, SEQ ID NO:2 oder SEQ ID NO:3 erhalten wird, wobei die abgeleitete Sequenz mindestens eine Region aus den folgenden Regionen umfasst: der Region im Bereich von der Aminosäure an Position 15 bis zur Aminosäure an Position 60, der Region im Bereich von der Aminosäure an Position 20 bis zur Aminosäure an Position 50, der Region im Bereich von der Aminosäure an Position 24 bis zur Aminosäure an Position 43, der Region im Bereich von der Aminosäure an Position 170 bis zur Aminosäure an Position 220, der Region im Bereich von der Aminosäure an Position 180 bis zur Aminosäure an Position 215 und der Region im Bereich von der Aminosäure an Position 190 bis zur Aminosäure an Position 211 der Sequenz SEQ ID NO:1, SEQ ID NO:2 oder SEQ ID NO:3; wodurch die Induktion einer Th2-Reaktion und/oder die Verringerung der Th1-Reaktion herbeigeführt wird, die jeweils durch die Produktion von IL-4, IL-5 oder IL-13 und die Produktion von IFN-gamma, von TNF oder von IL-1b gekennzeichnet sind; und
- wobei die homologe Sequenz eine homologe Sequenz einer der Sequenzen SEQ ID NO:1, SEQ ID NO:2 und SEQ ID NO:3 oder einer der Fragmente ist, und eine Identität von mindestens 80 % oder mindestens 85 % mit mindestens einer der folgenden Regionen der Sequenzen aufweist: der Region im Bereich von der Aminosäure an Position 15 bis zur Aminosäure an Position 60, der Region im Bereich von der Aminosäure an Position 20 bis zur Aminosäure an Position 50, der Region im Bereich von der Aminosäure an Position 24 bis zur Aminosäure an Position 43, der Region im Bereich von der Aminosäure an Position 170 bis zur Aminosäure an Position 220, der Region im Bereich von der Aminosäure an Position 180 bis zur Aminosäure an Position 215 und der Region im Bereich von der Aminosäure an Position 190 bis zur Aminosäure an Position 211 der Sequenz SEQ ID NO:1, SEQ ID NO:2 oder SEQ ID NO:3; oder mit der Gesamtheit einer der Sequenzen SEQ ID NO:1, SEQ ID NO:2 oder SEQ ID NO:3; wodurch die Induktion einer Th2-Reaktion und/oder die Verringerung der Th1-Reaktion herbeigeführt werden, die durch die Produktion von IL-4, IL-5 bzw. IL-13 und die Produktion von IFN-gamma, von TNF oder von IL-1b gekennzeichnet sind;
für seine Verwendung bei der vorbeugenden und/oder heilenden Behandlung einer chronischen entzündlichen Erkrankung, die mit einer Fehlregulierung des Immunsystems zusammenhängt, die eine Th1- und/oder Th17-Immunreaktion erzeugt, ausgewählt aus den entzündlichen Autoimmunkrankheiten, umfassend die Berger-Krankheit, Morbus Basedow, Hashimoto-Thyreoiditis, primäres Myxödem, Zöliakie, Colitis ulcerosa, Morbus Crohn, rheumatoide Arthritis, primäre biliäre Zirrhose, primäre sklerosierende Cholangitis, autoimmunhämolytische Anämien, Biermer-Anämie (perniziöse Anämie), Lupus erythematodes, CREST-Syndrom, Typ-1-Diabetes, Sklerodermie, Pemphigus vulgaris, bullöses Pemphigoid, erworbene Epidermolysis bullosa, Dermatitis herpetiformis, Myasthenie,
Lambert-Eaton-myasthene-Syndrom, Polymyositis, Gougerot-Sjögren-Syndrom, multiple Sklerose, rheumatoide Polyarthritis, Graves-Krankheit und Psoriasis.

2. Produkt zur Verwendung nach Anspruch 1, **dadurch gekennzeichnet, dass** das Protein durch die Sequenz SEQ ID NO:1 (28-GST-Glutathiontransferase von *Schistosoma haematobium*) dargestellt wird, wobei das Fragment ein Fragment des Proteins ist, das die Region im Bereich von der Aminosäure an Position 15 bis zur Aminosäure an Position 60 oder die Region im Bereich von der Aminosäure 20 bis zur Aminosäure 50 oder die Region im Bereich von der Aminosäure an Position 24 zu der Aminosäure an Position 43 und die Region im Bereich von der Aminosäure an Position 170 bis zur Aminosäure an Position 220 oder die Region im Bereich von der Aminosäure an Position 180 bis zur Aminosäure an Position 215 oder die Region im Bereich von der Aminosäure an Position 190 bis zur Aminosäure an Position 211 umfasst, wobei die abgeleitete Sequenz aus SEQ ID NO:1 erhalten wird und die homologe Sequenz eine Identität von mindestens 80 % oder mindestens 85 % mit der Sequenz SEQ ID NO:1 aufweist.

3. Produkt zur Verwendung nach Anspruch 2, **dadurch gekennzeichnet, dass** es das Expressionsprodukt einer Nukleotidsequenz ist, die:
- für das Protein 28KDa-Glutathion-S-Transferase-Protein, dargestellt durch SEQ ID NO:1; oder
- für ein Fragment des Proteins, dargestellt durch SEQ ID NO:1, wobei das Fragment mindestens die Region im Bereich von der Aminosäure an Position 15 bis zur Aminosäure an Position 60 oder die Region im Bereich von der Aminosäure an Position 20 bis zur Aminosäure an Position 50 oder die Region im Bereich von der Aminosäure an Position 24 bis zur Aminosäure an Position 43 und die Region im Bereich von der Aminosäure an Position 170 bis zur Aminosäure an Position 220 oder die Region im Bereich von der Aminosäure an Position 180 bis zur Aminosäure an Position 215 oder die Region im Bereich von der Aminosäure an Position 190 bis zur Aminosäure an Position 211 der Sequenz SEQ ID NO:1 umfasst oder daraus besteht; oder
- für eine der abgeleiteten Sequenzen; oder
- für eine der homologen Sequenzen kodiert;
wobei die Nukleotidsequenz der Sequenz SEQ ID NO: 4, die für SEQ ID NO:1 kodiert, entspricht.

4. Produkt zur Verwendung nach Anspruch 3, **dadurch gekennzeichnet, dass** es das Expressionsprodukt der kodierenden Sequenz SEQ ID NO:4 in *Saccharomyces cerevisiae* oder in *Escherichia coli* ist.

5. Produkt zur Verwendung nach einem der Ansprüche 1 bis 4 zur Verwendung bei einem Kind.

6. Pharmazeutische Zusammensetzung zur Verwendung nach einem der Ansprüche 1 und 5, wobei die Zusammensetzung als Wirkstoff ein Produkt, wie in einem der Ansprüche 1 bis 4 definiert, und einen pharmazeutisch annehmbaren Exzipienten umfasst.

7. Pharmazeutische Zusammensetzung zur Verwendung nach Anspruch 6 zur vorbeugenden und/oder heilenden Behandlung einer Krankheit, ausgewählt aus den entzündlichen Autoimmunkrankheiten, umfassend die Berger-Krankheit, Morbus Basedow, Hashimoto-Thyreoiditis, primäres Myxödem, Zöliakie, Colitis ulcerosa, Morbus Crohn, rheumatoide Arthritis, primäre biliäre Zirrhose, primäre sklerosierende Cholangitis, Autoimmunhämolytische Anämien, Biermer-Anämie (perniziöse Anämie), Lupus erythematodes, CREST-Syndrom, Typ-1-Diabetes, Sklerodermie, Pemphigus vulgaris, bullöses Pemphigoid, erworbene Epidermolysis bullosa, Dermatitis herpetiformis, Myasthenie, Lambert-Eaton-myasthene-Syndrom, Polymyositis, Gougerot-Sjögren-Syndrom, multiple Sklerose, rheumatoide Polyarthritis, Graves-Krankheit und Psoriasis und die möglichen pädiatrischen Formen dieser Krankheiten.

8. Zusammensetzung zur Verwendung nach einem der Ansprüche 1 oder 6, **dadurch gekennzeichnet, dass** sie ein Adjuvans, ausgewählt aus Aluminiumsalzen, umfasst.

9. Pharmazeutische Zusammensetzung zur Verwendung nach einem der Ansprüche 1, 6 und 7, **dadurch gekennzeichnet, dass** sie als Adjuvans Aluminiumhydroxid umfasst.

10. Zusammensetzung zur Verwendung nach Anspruch 9, **dadurch gekennzeichnet, dass** die Adjuvanskonzentration im Bereich von 0,5 mg/ml bis 2 mg/ml liegt.

11. Zusammensetzung zur Verwendung nach Anspruch 10, **dadurch gekennzeichnet, dass** die Adjuvanskonzentration im Bereich von 0,3 mg/ml bis 1 mg/ml liegt.

12. Zusammensetzung zur Verwendung nach Anspruch 10, **dadurch gekennzeichnet, dass** die Adjuvanskonzentration im Bereich von 220 µg/ml bis 280 µg/ml liegt.

13. Zusammensetzung zur Verwendung nach Anspruch 10, **dadurch gekennzeichnet, dass** die Adjuvanskonzentration im Wesentlichen gleich 250 µg/ml beträgt.

## Claims

1. Product selected from a protein, a fragment of said protein, a derived sequence and a homologous sequence of said protein, **characterized in that**:
- said protein comprises or is constituted by the 28 kDa glutathione S-transferase protein from a schistosome selected from *Schistosoma haematobium, Schistosoma mansoni, Schistosoma bovis* represented by the sequences SEQ ID NO: 1, SEQ ID NO: 2, and SEQ ID NO: 3, respectively;
- said fragment is a fragment of one of said proteins comprising at least one region from among the following regions: the region comprised from the amino acid in position 15 to the amino acid in position 60, the region comprised from the amino acid in position 20 to the amino acid in position 50, the region comprised from the amino acid in position 24 to the amino acid in position 43, the region comprised from the amino acid in position 170 to the amino acid in position 220, the region comprised from the amino acid in position 180 to the amino acid in position 215, the region comprised from the amino acid in position 190 to the amino acid in position 211 of the sequence SEQ ID NO: 1, SEQ ID NO: 2 or SEQ ID NO: 3;
and this inducing an immune response of type Th2 **characterized by** the production of IL-4, IL-5 or IL-13 by lymphocytes not producing IFN gamma;
and this inducing an immune response of type Th1, said immune response of type Th1 being **characterized by** the production of IFN gamma and of TNF by lymphocytes not producing IL-4 or IL-5, and the production of IL-1b by rat colic tissues;
- said derived sequence is derived from one of said sequences SEQ ID NO: 1, SEQ ID NO: 2 and SEQ ID NO: 3 or from one of said aforementioned fragments and is obtained in particular by suppression, substitution or addition of one or more amino acids, in the sequence SEQ ID NO: 1, SEQ ID NO: 2 or SEQ ID NO: 3, said derived sequence comprising at least one region from among the following regions: the region comprised from the amino acid in position 15 to the amino acid in position 60, the region comprised from the amino acid in position 20 to the amino acid in position 50, the region comprised from the amino acid in position 24 to the amino acid in position 43, the region comprised from the amino acid in position 170 to the amino acid in position 220, the region comprised from the amino acid in position 180 to the amino acid in position 215 and the region comprised from the amino acid in position 190 to the amino acid in position 211 of the sequence SEQ ID NO: 1, SEQ ID NO: 2 or SEQ ID NO: 3; this inducing the induction of a Th2 response and/or the decrease of the Th1 response; respectively **characterized by** the production of IL-4, IL-5 or IL-13, and the production of IFN gamma, of TNF or of IL-1b; and
- said homologous sequence is a homologous sequence of one of said sequences SEQ ID NO: 1, SEQ ID NO: 2 and SEQ ID NO: 3 or of one of said fragments, and has an identity of at least 80% and in particular of at least 85% with at least one of the following regions of said sequences: the region comprised from the amino acid in position 15 to the amino acid in position 60, the region comprised from the amino acid in position 20 to the amino acid in position 50, the region comprised from the amino acid in position 24 to the amino acid in position 43, the region comprised from the amino acid in position 170 to the amino acid in position 220, the region comprised from the amino acid in position 180 to the amino acid in position 215, and the region comprised from the amino acid in position 190 to the amino acid in position 211 of the sequence SEQ ID NO: 1, SEQ ID NO: 2 or SEQ ID NO: 3; or with the whole of one of the sequences SEQ ID NO: 1, SEQ ID NO: 2 or SEQ ID NO: 3; this inducing the induction of a Th2 response and/or the decrease of the Th1 response; respectively **characterized by** the production of IL-4, IL-5 or IL-13, and the production of IFN gamma, of TNF or of IL-1b;
for its use in the preventive and/or therapeutic treatment of a chronic inflammatory disease linked to an immune system disorder generating an immune response of type Th1/Th17 selected from the autoimmune inflammatory diseases comprising Berger's disease, Basedow's disease, Hashimoto's thyroiditis, primary myxoedema, coeliac disease, ulcerative colitis, Crohn's disease, rheumatoid arthritis, primary biliary cirrhosis, primary sclerosing cholangitis, autoimmune haemolytic anaemias, Biermer's anaemia (pernicious anaemia), lupus erythematosus, CREST syndrome, type 1 diabetes, scleroderma, pemphigus vulgaris, bullous pemphigoid, acquired epidermolysis bullosa, dermatitis herpetiformis, myasthenia, Lambert-Eaton myasthenic
syndrome, polymyositis, Goujerot-Sjögren syndrome, multiple sclerosis, rheumatoid arthritis, Graves' disease and psoriasis.

2. Product for its use according to claim 1, **characterized in that** said protein is represented by the sequence SEQ ID NO: 1 (28 GST glutathione transferase from *Schistosoma haematobium*)*,* said fragment is a fragment of said protein comprising the region comprised from the amino acid in position 15 to the amino acid in position 60, in particular the region from amino acid 20 to amino acid 50, preferably the region comprised from the amino acid in position 24 to the amino acid in position 43 and the region comprised from the amino acid in position 170 to the amino acid in position 220, in particular the region comprised from the amino acid in position 180 to the amino acid in position 215, preferably the region comprised from the amino acid in position 190 to the amino acid in position 211, said derived sequence is obtained from SEQ ID NO: 1 and said homologous sequence has an identity of at least 80% or at least 85% with the sequence SEQ ID NO: 1.

3. Product for its use according to claim 2, **characterized in that** it is the expression product of a nucleotide sequence coding:
- for the 28 kDa glutathione-S-transferase protein represented by SEQ ID NO: 1; or
- for a fragment of said protein represented by SEQ ID NO: 1, said fragment comprising or being constituted by at least the region comprised from the amino acid in position 15 to the amino acid in position 60, in particular the region comprised from the amino acid in position 20 to the amino acid in position 50, preferably the region comprised from the amino acid in position 24 to the amino acid in position 43 and the region comprised from the amino acid in position 170 to the amino acid in position 220, in particular the region comprised from the amino acid in position 180 to the amino acid in position 215, preferably the region comprised from the amino acid in position 190 to the amino acid in position 211 of the sequence SEQ ID NO: 1; or
- for one of said derived sequences; or
- for one of said homologuous sequences;
said nucleotide sequence corresponding to sequence SEQ ID NO: 4 coding for SEQ ID NO: 1.

4. Product for its use according to claim 3, **characterized in that** it is the expression product of said coding sequence SEQ ID NO: 4 in *Saccharomyces cerevisiae* or in *Escherichia coli.*

5. Product for its use according to any one of claims 1 to 4, for use in a child.

6. Pharmaceutical composition for its use according to any one of claims 1 et 5, said composition comprising, as active ingredient, a product as defined in any one of claims 1 to 4 and a pharmacologically acceptable excipient.

7. Pharmaceutical composition for its use according to claim 6, for the preventive and/or therapeutic treatment of a disease selected from the autoimmune inflammatory diseases comprising Berger's disease, Basedow's disease, Hashimoto's thyroiditis, primary myxoedema, coeliac disease, ulcerative colitis, Crohn's disease, rheumatoid arthritis, primary biliary cirrhosis, primary sclerosing cholangitis, autoimmune haemolytic anaemias, Biermer's anaemia (pernicious anaemia), lupus erythematosus, CREST syndrome, type 1 diabetes, scleroderma, pemphigus vulgaris, bullous pemphigoid, acquired epidermolysis bullosa, dermatitis herpetiformis, myasthenia, Lambert-Eaton myasthenic syndrome, polymyositis, Goujerot-Sjögren syndrome, multiple sclerosis, rheumatoid arthritis, Graves' disease and psoriasis and the possible paediatric forms of these diseases.

8. Composition for its use according to claim 6 or 7, **characterized in that** it comprises an adjuvant selected from the aluminium salts.

9. Pharmaceutical composition for its use according to any one of claims 1, 6 and 7, **characterized in that** it comprises as adjuvant aluminium hydroxide.

10. Composition for its use according to claim 9, **characterized in that** the concentration of adjuvant is from 0.5 mg/ml to 2 mg/ml.

11. Composition for its use according to claim 10, **characterized in that** the concentration of adjuvant is from 0.3 mg/ml to 1 mg/ml.

12. Composition for its use according to claim 10, **characterized in that** the concentration of adjuvant is from 220 µg/ml to 280 µg/ml.

13. Composition for its use according to claim 10, **characterized in that** the concentration of adjuvant is substantially equal to 250 µg/ml.
